# EUROPEAN PATENT APPLICATION

(11) **EP 4 789 664 A1**
(43) Date of publication of application: **12.08.2026**
(21) Application number: 25155345.9
(22) Date of filing: 10.02.2025
(51) Int. Cl.: A61K 31/136, A61K 31/165, A61K 31/167, A61K 31/222, A61K 31/277, A61K 31/352, A61K 31/4025, A61K 31/403, A61K 31/473, A61K 31/4741, A61K 31/498, A61K 31/65, A61K 45/06, A61P 31/14, A61P 31/18

(54) **NOVEL ANTIRETROVIRAL COMPOUNDS AND USES THEREOF**

(71) Applicant: UNIVERSITE DE BORDEAUX, 33000 Bordeaux (FR); Centre National de la Recherche Scientifique, 75016 Paris (FR)
(72) Inventor: METIFIOT, Mathieu, 33240 Saint André de Cubzac (FR); ANDREOLA, Marie-Aline, 33650 La Brède (FR); TORRES, Chloé, 33700 Mérignac (FR)
(74) Representative: Lecca, Patricia S.

(57) **Abstract**

This invention relates to anthraquinone; tetracycline; aporphine and pyrrole-derived compounds as antiretroviral therapy, pharmaceutical or veterinary compositions comprising said compounds and methods of using said compositions in the treatment and/or the prevention of disease or disorder associated with the activation of specific protein kinases, and more particularly in the treatment and/or the prevention of retroviral infections in mammals.

## Description

### FIELD OF THE INVENTION

This invention relates to anthraquinone, tetracycline, aporphine and pyrrole-derived compounds as antiretroviral therapy, as well as compositions, such as pharmaceutical and/or veterinary compositions comprising said compounds and methods of using said compositions in the treatment and/or the prevention of disease or disorder associated with the activation of specific protein kinases, and more particularly in the treatment and/or the prevention of retroviral infections in mammals.

### BACKGROUND OF THE INVENTION

Retroviruses are viruses whose single-stranded RNA genome is reverse transcribed into double-stranded DNA then integrated into the genome of the infected cell. In addition to viral enzymes responsible for essential catalytic activities such as the reverse transcriptase (RT), numerous viral proteins (e.g. CA, VPR) and cellular proteins (e.g. LEDGF/p75, CPSF6), the retrovirus integrase (IN) is thus crucial to this process. Of the three virally encoded enzymes, IN has proven to be the most difficult to target. This protein from the polynucleotidyl transferase family is structurally related to RNase H, which raised concerns about its "drugability" without toxicity. IN is a 32 kDa protein (288 amino acids) composed of three domains, all involved in DNA binding and oligomerization of the enzyme. The core domain (50-212) harbors a catalytic triad DDE that binds two metal cofactors (Mg2+) responsible for the two successive transesterification reactions needed to perform integration. After binding the viral DNA long terminal repeats (LTRs), the terminal dinucleotide at the 3' ends are cleaved (3'-processing, 3'-P). Then, these newly created 3'-OH extremities are used to attack the target DNA (strand transfer, ST) leading to the integration of the provirus into the host genome. Nonetheless, a post-integration repair is required to restore DNA integrity that is supported by cellular processes (5' overhang recession and gap filling).

From the therapeutic point of view, IN has been validated as a target by the introduction of raltegravir (RAL) to the clinic in 2007. This first in-class drug is a highly specific catalytic inhibitor binding at the interface of the enzyme, the DNA substrate and chelating the two metal cations in the active site. Because RAL and other IN active site inhibitors exhibit a relative selectivity for the inhibition of ST over 3'-P, this class of molecules is often referred to as INSTIs. However, resistance to INSTIs aroused with mutations in the IN-coding region leading to a reduced susceptibility to this class of drugs.

Because of the ability of HIV-1 to constantly evolve and adapt in its host, the emergence of resistance mutations affects all classes of antiretrovirals, there is a need for new strategies aiming to target IN out of its active site should enable to overcome this resistance phenomenon.

Besides its canonical role in integration, IN has non-catalytic functions during replication. It has been implicated in i) the reverse transcription step thanks to its interaction with RT, ii) nuclear translocation of the pre-integration complex (PIC) through its NLS and interaction with nuclear pore complex proteins and transport-associated proteins, and iii) virus packaging and morphology, including interactions with the viral RNA and capsid. Altogether, this emphasizes the crucial role of IN during HIV replication, not only because of its catalytic activity but also through its numerous protein-protein interactions. Among them, the most described partner of IN is the transcription factor lens epithelium-derived growth factor (LEDGF)/p75. LEDGF interacts with IN (core domain) through its integrase binding domain (amino acid residues 347-429) at a dimer interface, promoting IN oligomerization. Because of its chromatin binding activity, LEDGF tethers IN and subsequently the PIC to the integration site. Altogether, the cellular integration activity is highly dependent on the presence of LEDGF and knocking-down or out the protein resulted in a dramatic reduction in integration efficacy and viral replication. Accordingly, molecules targeting the LEDGF binding site of IN were developed (LEDGINs). Unexpectedly at the time, targeting the LEDGF binding site of IN resulted in an allosteric inhibition of IN catalytic activities and induced morphologic aberrations of the newly produced viral particles (ALLINIs). While ALLINIs are still actively studied, none of them has yet reached the clinic.

In parallel, other protein-protein interactions have been explored. Among them, IN interacts with Ku70, part of the DNA-PK complex and involved in DNA damage repair (non-homologous end Joining, NHEJ). A virtual screening enabled the identification of a novel series of compounds based on the pyrrolo[1,2-a]quinolone scaffold. Unfortunately, while le lead compound (s17) is able to inhibit the IN-Ku70 interaction with an IC50 of 13 ± 2 µM, none of the derivatives tested to date exhibited enhanced activity.

GCN2 (General Control Nonderepressible 2) is a serine/threonine-protein kinase which has been identified as a cellular partner of the retrovirus integrase. GCN2 is one of the four eIF2α kinases involved in the integrated stress response (ISR). HIV-1 infection activates GCN2 that leads to transient translation arrest in the cell. GCN2 not only interacted with IN but also phosphorylated the enzyme on its C-terminal domain. Mutation of residue S255 increased viral DNA integration and GCN2-depleted cells displayed higher levels of replication.

The present invention addresses the long felt need to provide inhibitors of protein-protein interactions initiated by protein-sensing kinases integrated response to control viral infections and could be used for other conditions involving ISR (response innate immune system). It also takes advantage of the specificity of recognition of substrate to interfere with phosphorylation by ISR kinases (in particular GCN2) by minimizing the risk of inhibition of other cellular kinases.

### SUMMARY OF THE INVENTION

The present invention relates to compounds derived from anthraquinones, tetracyclines, aporphines and/or pyrroles capable of modulating (e.g., inhibiting or activating) protein kinase selected among GCN2 (General Control Nonderepressible 2), PKR (Protein Kinase R), HRI (Heme-regulated inhibitor), PERK (PKR-like endoplasmic reticulum kinase). Preferably, the present invention relates to novel derivatives capable of inhibiting IN-GCN2 interactions which are approximately two times more active than the parent compounds.

The present invention also relates to compositions, including pharmaceutical and/or veterinary compositions comprising said novel derivative compounds, salts, enantiomers, stereoisomers, or tautomers thereof, and a pharmaceutically or a veterinary acceptable carrier or excipient.

The present invention further relates to the use of said novel derivative compounds as antiretroviral therapy, and more precisely for use in a method of treating and/or preventing retroviral infection in a mammal in need thereof.

The present invention finally relates said novel derivative compounds for use in a method of inhibiting and/or in a method of treating and/o preventing a disease or disorder associated with the activation of a protein kinase selected among GCN2, PKR (Protein Kinase R), HRI (Heme-regulated inhibitor), PERK (PKR-like endoplasmic reticulum kinase). Preferably, the present invention relates to the use of said novel compounds in a method of inhibiting GCN2 kinase or PERK kinase and/or a method of treating and/or preventing GCN2 associated disease and/or a PERK associated disease in a subject in need thereof.

### BRIEF DESCRIPTION OF THE FIGURES

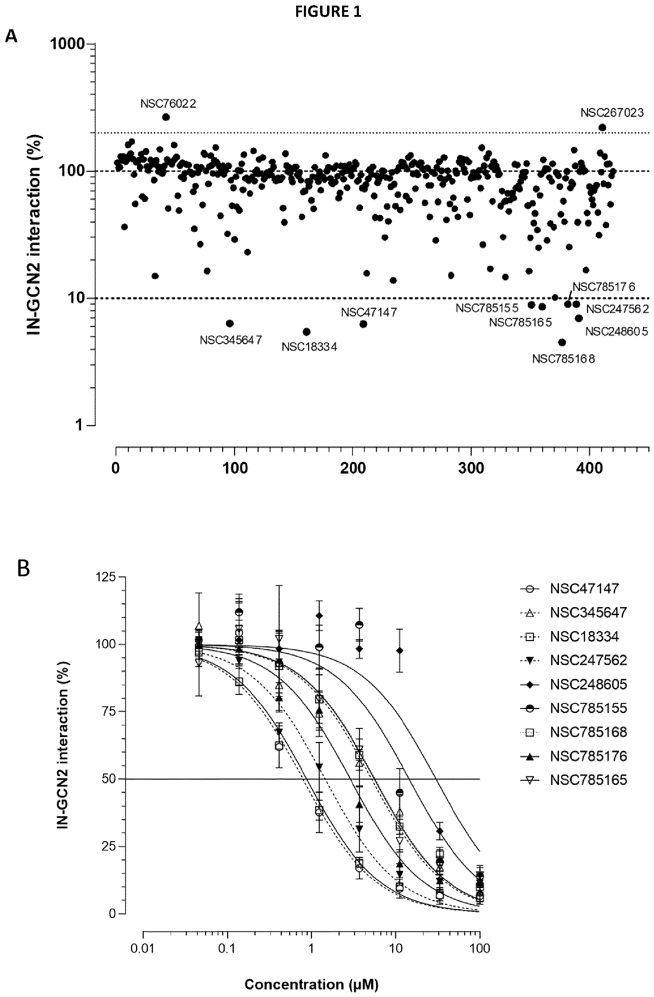

**Figures 1A****-B: (A)** corresponds to a graph showing the effect of compounds from natural products on the IN-GCN2 interaction measured by AlphaLISA. Each compound was tested at a unique dose of 100 µM in a single replicate. Signals were normalized to DMSO control. Thresholds were set at 10% signal (green dashed line) to select molecules inhibiting at least 90% of the control signal (green dots) and 200% (red dotted line) to select potential stimulators (red dots). **(B)** corresponds to dose-response curves of the nine inhibitors on the IN-GCN2 interaction. Mean and standard deviation were calculated from three replicates in three independent experiments.
**Figure 2****:** shows the chemical structure of selected inhibitors.
**Figure 3****:** shows the chemical structure of selected inhibitors.
**Figure 4** corresponds to dose-response curves for the inhibition of the IN-GCN2 interaction by tetracyclines. IC50 and SD values are provided from at least three independent experiments.
**Figure 5****:** corresponds to dose-response curves for the inhibition of the IN-GCN2 interaction by anthracyclines. IC50 and SD values are provided from at least three independent experiments.
**Figure 6****:** corresponds to dose-response curves for the inhibition of the IN-GCN2 interaction by anthraquinones. IC50 and SD values are provided from at least three independent experiments.
**Figure 7****:** corresponds to dose-response curves for the inhibition of the IN-GCN2 interaction by aporphines. IC50 and SD values are provided from at least three independent experiments.
**Figure 8****:** shows representative gels of a phosphorylation assay in the presence of NSC785155, NSC345647 and NSC18334. The phosphorylation of GCN2 and IN was monitored in the presence of DMSO or a decreasing concentration of compound (from 10 µM to 4.6 nM). It thus shows the effect of chemical modulators of the IN-GCN2 interaction.
**Figure 9****:** corresponds to graphs showing the inhibition of GCN2's activation and IN's phosphorylation by selected compounds. Gels were submitted to autoradiography and quantified. Means and non-linear regression resulted from at least two independent determinations.
**Figure 10****:** is a table showing cytotoxicity and antiretroviral activity of compounds of the present invention. ND = "not determined".
**Figure 11****:** are graphs showing the cytotoxicity and antiretroviral activity of compounds of the present invention.

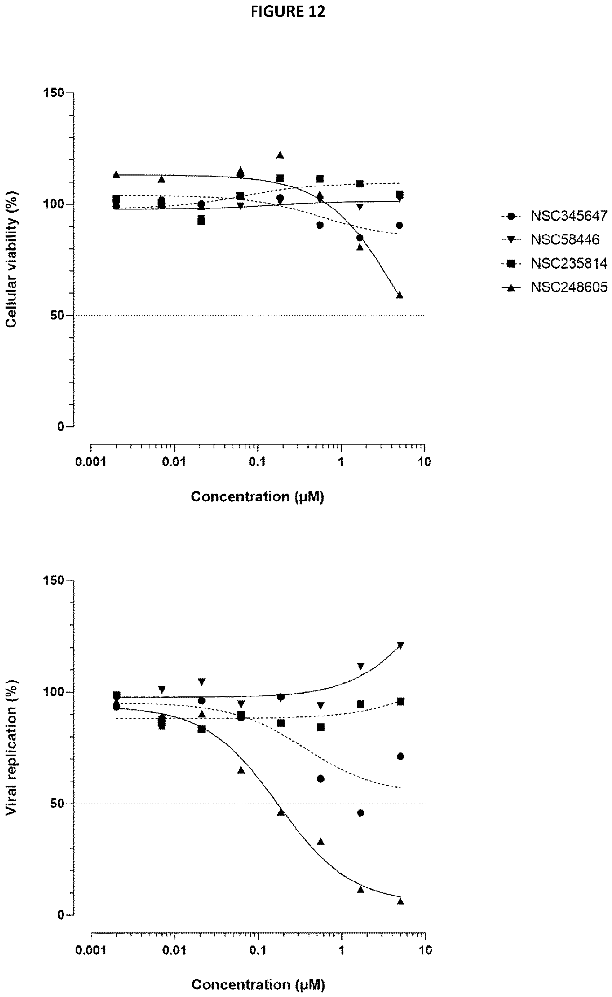

**Figure 12****:** are graphs showing the cytotoxicity and antiretroviral activity of compounds of the present invention.

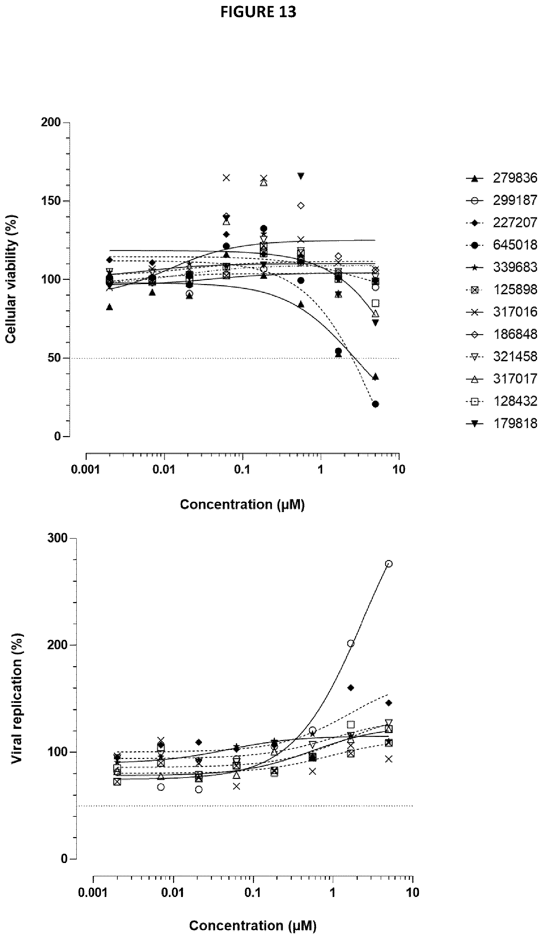

**Figure 13****:** are graphs showing the cytotoxicity and antiretroviral activity of compounds of the present invention.

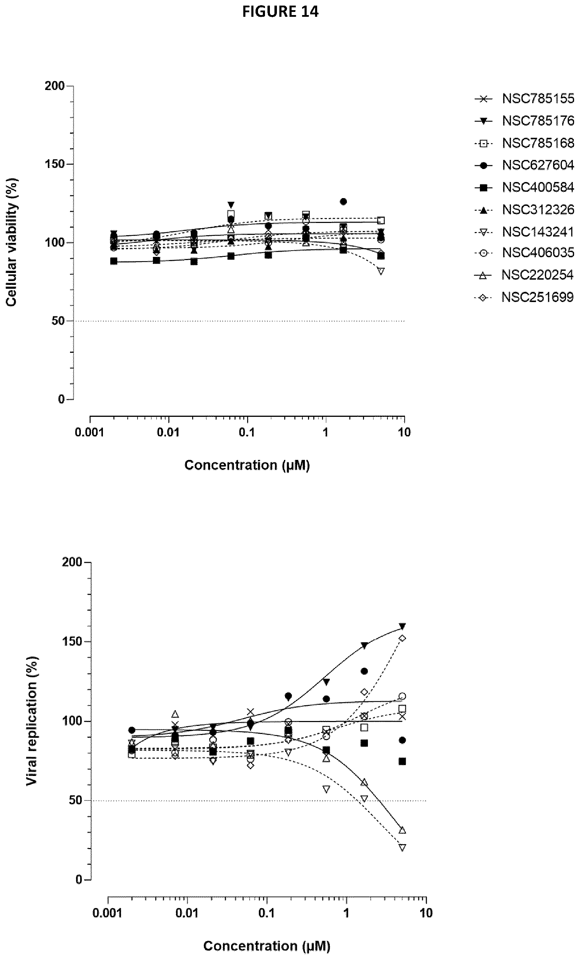

**Figure 14****:** are graphs showing the cytotoxicity and antiretroviral activity of compounds of the present invention.

### DETAILED DESCRIPTION OF THE INVENTION

The features and other details of the disclosure are more particularly described below. Certain terms employed in the specification, examples and appended claims are collected here. These definitions should be read in light of the remainder of the disclosure and as understood by a person of skill in the art. Unless defined otherwise, all technical and scientific terms used herein have the same meaning as commonly understood by a person of ordinary skill in the art.

The definitions set forth in this application are intended to clarify terms used throughout this application. Unless defined otherwise, all technical and scientific terms used herein have the same meaning as is commonly understood by one of skill in the art to which the subject matter herein belongs. As used in the specification and the appended claims, unless specified to the contrary, the following terms have the meaning indicated in order to facilitate the understanding of the present disclosure.

When a 'bond' to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents, positions of substituents and/or variables are permissible if such combinations result in stable compounds.

As used herein, the terms "optional" or "optionally" mean that the subsequently described event or circumstance may occur or may not occur, and that the description includes instances where the event or circumstance occurs as well as instances in which it does not. For example, "optionally substituted alkyl" refers to the alkyl may be substituted as well as where the alkyl is not substituted.

As used herein, the term "optionally substituted" refers to the replacement of one to six hydrogen atoms in a given structure with the radical of a specified substituent including, but not limited to: hydroxy, hydroxyalkyl, alkoxy, halogen, alkyl, aryl, cycloalkyl, heterocyclyl, amino, aminoalkyl, cyano, haloalkyl, haloalkoxy, -OC(=O)-CH2-Oalkyl. Preferably, "optionally substituted" refers to the replacement of one to four hydrogen atoms in a given structure with the substituents mentioned above. More preferably, one to three hydrogen atoms are replaced by the substituents as mentioned above. It is understood that the substituent can be further substituted.

As used herein, the term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this application, the heteroatoms such as nitrogen may have hydrogen substituents and/or any permissible substituents of organic compounds described herein which satisfy the valences of the heteroatoms. If a substituent is itself substituted with more than one group, it is understood that these multiple groups may be on the same carbon or on different carbons, so long as a stable structure result. Substituents can include any substituents described herein, for example, such substituents, if not otherwise specified, can include, for example, a halogen, a hydroxy, a carbonyl (such as a carboxyl, an alkoxycarbonyl, a formyl, or an acyl), an alkoxy, an amino, an amido, an imine, a cyano, a sulfonyl, a heterocyclyl, an aralkyl, a heteroaralkyl, or an aromatic or heteroaromatic moiety. It will be understood by those skilled in the art that substituents can themselves be substituted, if appropriate.

For instance, the substituents of a substituted alkyl may include substituted and unsubstituted forms of amino, amido, sulfonyl and as well as ethers, carbonyls (including carboxylates, and esters), -CF3, -CN and the like. Unless specifically stated as "unsubstituted," references to chemical moieties herein are understood to include substituted variants. For example, reference to an "aryl" group or moiety implicitly includes both substituted and unsubstituted variants.

As used herein, the term "alkyl" refers to a straight chained or branched non- aromatic hydrocarbon which is completely saturated. Typically, a straight chained or branched alkyl group has from 1 to about 20 carbon atoms, preferably from 1 to about 10, e.g., may be C1-C10alkyl or e.g., C1-C6alkyl unless otherwise defined. Examples of straight chained and branched alkyl groups include, but are not limited to, methyl, ethyl, 1-propyl (n- propyl), 2-propyl, n-butyl, sec-butyl, tertbutyl, 1-pentyl, 2-pentyl, 3-pentyl, neo-pentyl, 1- hexyl, 2-hexyl, 3-hexyl, 1-heptyl, 2-heptyl, 3-heptyl, 4-heptyl, 1-octyl, 2-octyl, 3-octyl or 4- octyl and the like. Moreover, the term "alkyl" used throughout the specification, examples, and claims is intended to include both "unsubstituted alkyls" and "substituted alkyls", the latter of which refers to alkyl moieties having substituents replacing a hydrogen on one or more carbons of the hydrocarbon backbone. The "alkyl" group may be optionally substituted.

The term "alkenyl" as used herein refers to an unsaturated straight or branched hydrocarbon having at least one carbon-carbon double bond. Exemplary alkenyl groups include, but are not limited to, a straight or branched group of 2-6 or 3-4 carbon atoms, referred to herein as C2-C6alkenyl, and C3-C4alkenyl, respectively. Exemplary alkenyl groups include, but are not limited to, vinyl, allyl, butenyl, pentenyl, etc. The term "alkynyl" as used herein refers to an unsaturated straight or branched hydrocarbon having at least one carbon-carbon triple bond. Exemplary alkynyl groups include, but are not limited to, straight or branched groups of 2-6, or 3-6 carbon atoms, referred to herein as C2-C6alkynyl, and C3-C6alkynyl, respectively. Exemplary alkynyl groups include, but are not limited to, ethynyl, propynyl, butynyl, pentynyl, hexynyl, methylpropynyl, etc.

As used herein, the term "alkoxy" refers to a straight or branched, saturated aliphatic (alkyl) hydrocarbon radical bonded to an oxygen atom that is attached to a core structure. Preferably, alkoxy groups have one to six carbon atoms, *i.e*.*,* may be C₁-C₆ alkoxy. Examples of alkoxy groups include but are not limited to methoxy, ethoxy, propoxy, isopropoxy, butoxy, isobutoxy, tert-butoxy, pentoxy, 3-methyl butoxy and the like.

As used herein, the term "aryl" includes substituted or unsubstituted single- ring aromatic groups in which each atom of the ring is carbon. Preferably the ring is a 5- to 7- membered ring, more preferably a 6-membered ring. The term "aryl" also includes polycyclic ring systems having two or more cyclic rings in which two or more carbons are common to two adjoining rings (fused rings) wherein at least one of the rings is aromatic. e.g., the other cyclic rings can be cycloalkyls, cycloalkenyls, cycloalkynyls, aryls, heteroaryls, and/or heterocyclyls.

The term "fused" means that the second ring is attached or formed by having two adjacent atoms in common with the first ring. The term "fused" is equivalent to the term "condensed". Examples of aryl groups include but are not limited to phenyl, naphthyl, phenanthryl, phenol, aniline, indanyl, dihydrobenzofuranyl, dihydroisobenzofuranyl, indolinyl, isoindolinyl, and the like. Unless otherwise specified, aryl groups described herein may be optionally substituted.

As used herein, the terms "polycyclyl", "polycycle", and "polycyclic" refer to two or more rings (e.g., cycloalkyls, cycloalkenyls, cycloalkynyls, aryls, heteroaryls, and/or heterocyclyls) in which one or more atoms are common to two adjoining rings, e.g., the rings are "fused rings". Each of the rings of the polycycle can be substituted or unsubstituted. In certain embodiments, each ring of the polycycle contains from 3 to 10 atoms in the ring, preferably from 5 to 7.

As used herein, the term "cyano" refers to -CN group.

As used herein, the term "hydroxy" or "hydroxyl" refers to -OH group.

As used herein, the term "halo" or "halogen" alone or in combination with other term(s) means chloro, fluoro, bromo, and iodo.

As used herein, the term "heteroatom" refers an atom of any element other than carbon or hydrogen. Exemplary heteroatoms are nitrogen (N), oxygen (O), sulfur (S), and silicon (Si).

As used herein, the terms "heterocyclyl", "heterocycloalkyl", "heterocycle", and "heterocyclic" refer to a non-aromatic, saturated or partially saturated, including monocyclic, polycyclic (e.g., bicyclic, tricyclic) bridged, or fused, ring system of 3 to 15 member having at least one heteroatom or heterogroup selected from O, N, S, S(O), S(O)2, NH or C(O) with the remaining ring atoms being independently selected from the group consisting of carbon, oxygen, nitrogen, and sulfur. Examples of "heterocycloalkyl" include, but are not limited to azetidinyl, oxetanyl, imidazolidinyl, pyrrolidinyl, oxazolidinyl, thiazolidinyl, pyrazolidinyl, tetrahydrofuranyl, piperidinyl, piperazinyl, tetrahydropyranyl, morpholinyl, thiomorpholinyl, 1,4-dioxanyl, dioxidothiomorpholinyl, oxapiperazinyl, oxapiperidinyl, tetrahydrofuryl, tetrahydropyranyl, tetrahydrothiophenyl, dihydropyranyl, indolinyl, indolinylmethyl, 2-azabicyclo[2.2.2]octanyl, azocinyl, chromanyl, xanthenyl and N-oxides thereof. Attachment of a heterocycloalkyl substituent can occur via either a carbon atom or a heteroatom. A heterocycloalkyl group can be optionally substituted with one or more suitable groups by one or more aforesaid groups. Preferably "heterocycloalkyl" refers to 5- to 6-membered ring selected from the group consisting of azetidinyl, oxetanyl, imidazolidinyl, pyrrolidinyl, oxazolidinyl, thiazolidinyl, pyrazolidinyl, tetrahydrofuranyl, piperidinyl, piperazinyl, tetrahydropyranyl, morpholinyl, thiomorpholinyl, 1,4-dioxanyl and N-oxides thereof. More preferably, "heterocycloalkyl" includes azetidinyl, pyrrolidinyl, morpholinyl and piperidinyl. Heterocycloalkyl are optionally substituted by one or more aforesaid groups.

The term "pharmaceutically acceptable salt(s)" as used herein refers to salts of acidic or basic groups that may be present in compounds used in the compositions. Compounds included in the present compositions that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, including, but not limited to, malate, oxalate, chloride, bromide, iodide, nitrate, sulfate, bisulfate, phosphate, acid phosphate, isonicotinate, acetate, lactate, salicylate, citrate, tartrate, oleate, tannate, pantothenate, bitartrate, ascorbate, succinate, maleate, gentisate, fumarate, gluconate, glucaronate, saccharate, formate, benzoate, glutamate, methanesulfonate, ethanesulfonate, benzenesulfonate, p-toluenesulfonate and pamoate (i.e., 1,1'-methylene-bis-(2- hydroxy-3-naphthoate)) salts. Compounds included in the present compositions that are acidic in nature are capable of forming base salts with various pharmacologically acceptable cations. Examples of such salts include alkali metal or alkaline earth metal salts, particularly calcium, magnesium, sodium, lithium, zinc, potassium, and iron salts. Compounds included in the present compositions that include a basic or acidic moiety may also form pharmaceutically acceptable salts with various amino acids. The compounds of the disclosure may contain both acidic and basic groups; for example, one amino and one carboxylic acid group. In such a case, the compound can exist as an acid addition salt, a zwitterion, or a base salt.

The compounds of the disclosure may contain one or more chiral centers and, therefore, exist as stereoisomers. The term "stereoisomers" when used herein consist of all enantiomers or diastereomers. These compounds may be designated by the symbol "R" or "S" depending on the configuration of substituents around the stereogenic carbon atom, but the skilled artisan will recognize that a structure may denote a chiral center implicitly. These compounds may also be designated by "(+)" and "(-)" based on their optical rotation properties. The presently described compounds encompass various stereoisomers of these compounds and mixtures thereof. Mixtures of enantiomers or diastereomers may be designated by the symbol "(±)" in nomenclature, but the skilled artisan will recognize that a structure may denote a chiral center implicitly.

Individual enantiomers and diastereomers of the disclosed compounds can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, (3) direct separation of the mixture of optical enantiomers on chiral liquid chromatographic columns or (4) kinetic resolution using stereoselective chemical or enzymatic reagents. Racemic mixtures can also be resolved into their component enantiomers by well-known methods, such as chiral-phase liquid chromatography or crystallizing the compound in a chiral solvent. Stereoselective syntheses, a chemical or enzymatic reaction in which a single reactant forms an unequal mixture of stereoisomers during the creation of a new stereocenter or during the transformation of a pre-existing one, are well known in the art.

Stereoselective syntheses encompass both enantio- and diastereoselective transformations and may involve the use of chiral auxiliaries.

The term "pharmaceutical composition" as used herein refers to a composition comprising at least one compound as disclosed herein formulated together with one or more pharmaceutically acceptable carriers. The term "veterinary composition" as used herein refers to a composition comprising at least one compound as disclosed herein formulated together with one or more veterinary acceptable carriers.

The term "acceptable carrier" or "acceptable excipient" as used herein refers to any and all solvents, dispersion media, coatings, isotonic and absorption delaying agents, and the like, that are compatible with pharmaceutical or veterinary administration. The use of such media and agents for pharmaceutic or veterinary active substances is well known in the art. The compositions may also contain other active compounds providing supplemental, additional, or enhanced therapeutic functions.

In the present specification, the term "therapeutically effective amount" means the amount of the subject compound that will elicit the biological or medical response of a tissue, system or animal, (e.g., mammal or human) that is being sought by the researcher, veterinarian, medical doctor or other clinician. The compounds described herein are administered in therapeutically effective amounts to treat a disorder. "Treating" includes any effect, e.g., lessening, reducing, modulating, or eliminating, that results in the improvement of the condition, disease, disorder, and the like.

A "combination therapy" is a treatment that includes the administration of two or more therapeutic agents, e.g., a compound of Formula I, IA, II and III with another therapeutic agent, to a patient in need thereof. "Disease," "disorder," and "condition" are used interchangeably herein.

"Individual," "patient," or "subject" are used interchangeably and include any animal, including mammals, preferably mice, rats, other rodents, rabbits, dogs, cats, swine, cattle, sheep, horses, or primates, and most preferably humans. The compounds described herein can be administered to a mammal, such as a human, but can also be administered to other mammals such as an animal in need of veterinary treatment, e.g., domestic animals (e.g., dogs, cats, and the like), farm animals (e.g., cows, sheep, pigs, horses, and the like) and laboratory animals (e.g., rats, mice, guinea pigs, and the like).

The present invention thus relates to novel chemical compounds for use as antiviral therapy.

More precisely, the chemical compounds according to the present invention relates to a GCN2 inhibitor compound chosen among a compound having the following general formula (I) or (IA): 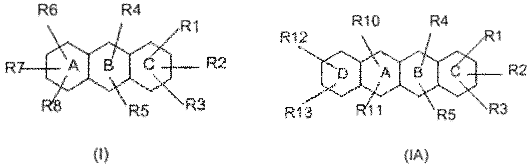
wherein rings A, B, C and D are selected from a six-membered saturated or unsaturated optionally substituted carbocylic ring, optionally containing one to two heteroatoms, selected from O, N or S;
wherein R₁, R₂ and R₃ are each independently selected from hydrogen, hydroxy, carbonyl, optionally substituted C₁-C₆ alkyl, a polycyclic saturated or unsaturated ring containing one to two heteroatoms, selected from O, N or S, substituted with one or more substituents independently selected from H, OH, carbonyl, optionally substituted C₁-C₆ alkyl; wherein the alkyl may be substituted with halogen, alkoxy, carbonyl, cyano, NO2, C(O)OR, or -OC(O)R, wherein R is any group selected from H or C₁-C₆ alkyl;
wherein R₄ and R₅ are each independently selected from H, carbonyl or OH;
wherein each of the groups R₆, R₇ and R₈ are independently selected from H, OH, carbonyl, optionally substituted C₁-C₆ alkyl, wherein the alkyl may be substituted with halogen, alkoxy, carbonyl, cyano, NO2, C(O)OR or -OC(O)R, and R is as defined above; or a polycyclic saturated or unsaturated ring containing C10-C18 ring atoms optionally containing one to two heteroatoms, selected from O, N or S; substituted with one or more substitutions independently selected from H, OH, carbonyl, optionally substituted C₁-C₆ alkyl;
wherein each of the groups R₁₀ and R₁₁ are independently selected from H, OH, carbonyl, optionally substituted with C₁-C₆ alkyl, wherein the alkyl may be substituted with halogen, alkoxy, carbonyl, cyano, NO2, C(O)OR or -OC(O)R, and R is as defined above;
wherein each of the groups R₁₂ and R₁₃ are independently selected from H, OH, carbonyl C₁-C₆ alkoxy, C(O)OR or -OC(O)R, R is as defined above, optionally substituted C₁-C₆ alkyl, wherein the alkyl may be substituted with halogen, alkoxy, carbonyl, cyano, NO2; or a group of formula (IV): 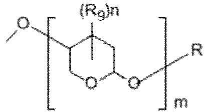
R₉ being selected from H, CO, C₁-C₆ alkyl, N(R)₂, wherein R is as defined above; and n is any integer chosen between 1-4; and m is any integer chosen between 1-4;
or a compound having the following general formula (II): 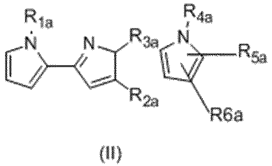
wherein R₁ₐ and R₄ₐ is hydrogen, phenyl, C₁-C₂₀ alkyl or C₂-C₂₀ alkenyl, wherein the alkyl and alkenyl groups are unsubstituted or substituted by 1 to 3 substituents chosen independently from halogen, alkoxy, hydroxy, aryl and aryloxy;
wherein R₂ₐ is hydrogen, C₁-C₆ alkyl, cyano, carboxy or (C₁-C₆ alkoxy) carbonyl;
wherein R₃ₐ is C₂-C₂₀ alkenyl;
wherein each of R₅ₐ and R₆ₐ, may be the same or different, independently represents hydrogen, C₁-C₂₀ alkyl, C₂-C₂₀ alkenyl, C₂-C₂₀ alkanoyl, C₃-C₂₀ alkenoyl, phenyl, wherein the alkanoyl, alkenoyl, alkyl and alkenyl groups are unsubstituted or substituted by 1 to 3 substituents chosen independently from halogen, C₁-C₆ alkoxy, hydroxy, aryl, aryloxy, cyano, carboxy, (C₁-C₆ alkoxy) carbonyl , aryloxy or aryl; or
wherein R₅ₐ and R₆ₐ, taken together with adjacent carbon ring atoms to which they are linked, form a C₄ - C₁₂ completely or partially saturated or unsaturated cyclic ring optionally contains one or more heteroatoms selected from O, S or N which is unsubstituted or substituted by C₁-C₁₂ alkyl, C₂-C₁₂ alkenyl, wherein the alkyl, and alkenyl groups are in turn unsubstituted or substituted by halogen, C₁-C₆ alkoxy, hydroxy, cyano, carboxy, (C₁-C₆ alkoxy) carbonyl, aryloxy or aryl; or a pharmaceutically acceptable salt thereof;
or a compound having the following general formula (III): 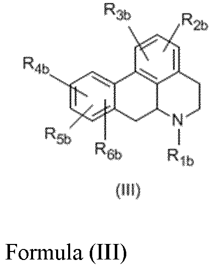
wherein R_{1b} is selected from H, C₁-C₂₀ alkyl, C₂-C₂₀ alkenyl, C₂-C₂₀ alkanoyl, C₃-C₂₀ alkenoyl, phenyl, wherein the alkanoyl, alkenoyl, alkyl and alkenyl groups are unsubstituted or substituted by 1 to 3 substituents chosen independently from halogen, C₁-C₆ alkoxy, hydroxy, aryl, aryloxy, cyano, carboxy, (C₁-C₆ alkoxy) carbonyl, aryloxy or aryl;
wherein each of R_{2b} and R_{3b}, which are the same or different, independently represents H, OH, carbonyl, optionally substituted C₁-C₆ alkyl; wherein the alkyl may be substituted with halogen, alkoxy, carbonyl, cyano, NO2, C(O)OR or -OC(O)R, wherein R is any group selected from H or C₁-C₆ alkyl;
or wherein R_{2b} and R_{3b}, taken together with adjacent carbon ring atoms which they are linked, form a C4 - C12 completely or partially saturated or unsaturated cyclic ring optionally contains one or more heteroatoms selected from O, S or N which is unsubstituted or substituted by C₁-C₁₂ alkyl, C₂-C₁₂ alkenyl, wherein the alkyl, and alkenyl groups are in turn unsubstituted or substituted by halogen, C₁-C₆ alkoxy, hydroxy, cyano, carboxy, (C₁-C₆ alkoxy) carbonyl, aryloxy or aryl;
wherein each of the groups R_{4b}, R5b and R_{6b} are independently selected from H, OH, halogen, C₁-C₆ alkoxy, cyano, carboxy, (C₁-C₆ alkoxy) carbonyl, aryloxy, aryl or NR'R"; R' and R‴ being the same or distinct, independently represents H, OH, carbonyl, optionally substituted C₁-C₆ alkyl, cyano, carboxy, (C₁-C₆ alkoxy) carbonyl, aryloxy or aryl;
or a pharmaceutically acceptable salt thereof.

Compounds of the present invention may be obtained, *inter alia,* from the National Cancer Institute through the National Cancer Institute Developmental Therapeutics Program (NCI DTP, NIH). They are designated below by their chemical names, chemical formula, and their respective National Service Center (NSC) identifiers as assigned by the National Center Institute at the DTP repositories. These identifiers are used in the Developmental Therapeutics Program (DTP) repository to track and catalog compounds for research and development.

Compounds of the present invention may be obtained *inter alia* from the National Cancer Institute were obtained through the National Cancer Institute Developmental Therapeutics Program (NCI DTP, NIH). They are designated below by their chemical names, chemical formula, and by their respective According to first embodiment, compounds according to the present invention may have the following general formula (I): 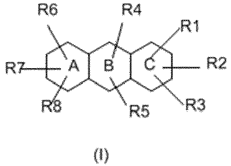 wherein rings A, B, and C in compound of formula (I) may correspond to an anthracene or an anthraquinone ring as shown below:

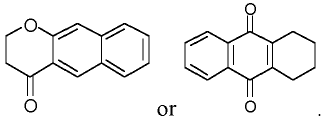

Compounds of formula (I) according to the first embodiment of the present invention may be anthracene derivatives and may be selected among compounds having the following formula: 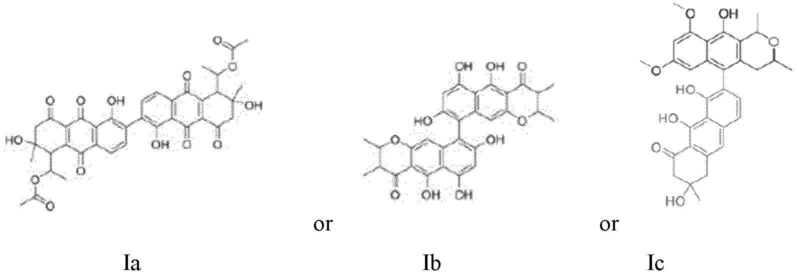 or a pharmaceutically acceptable salt, enantiomer, stereoisomer, or tautomer thereof.

Compounds of formula I according to the first embodiment of the present invention may also be anthraquinone derivatives and may be selected from the group consisting of compounds listed in the following Table 1.

**Table 1**

| | |
|---|---|
| I-2a | 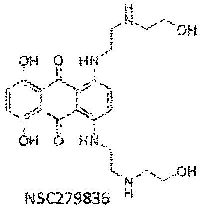 |
| NSC279836 | |
| Mitoxantrone | |
| 1,4-dihydroxy-5,8-bis[[2-[(2-hydroxyethyl)amino]ethyl]amino]-9,10-anthracenedione | |
| I-2d | 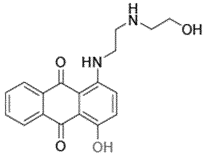 |
| NSC299187 | |
| 1-hydroxy-4-[[2-[(2-hydroxyethyl)amino]ethyl]amino]-9,10-anthracenedione | |
| I-2e | 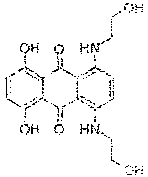 |
| NSC227207 | |
| 1,8-bis[(2-hydroxyethyl)amino]anthraquinone | |
| I-2f | 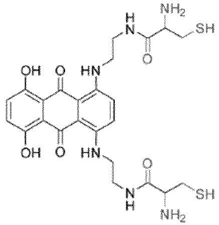 |
| NSC645018 | |
| 1,4-bis(N-Cysteinyldiethylamino)-5,8-dihydroxy anthraquinone | |
| I-2g | 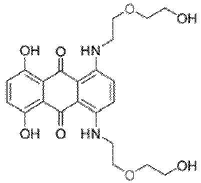 |
| NSC339683 | |
| 1,4-dihydroxy-5,8-bis[[2-(2-hydroxyethoxy)ethyl]amino]-9,10-anthracenedione | |
| I-2h | 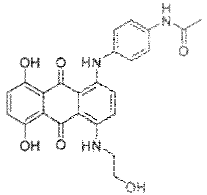 |
| NSC125898 | |
| N-[4-[[5,8-dihydroxy-4-(2-hydroxyethylamino)-9,10-dioxoanthracen-1-yl]amino]phenyl]acetamide | |
| I-2i | 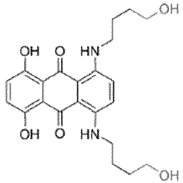 |
| NSC317016 | |
| 1,4-dihydroxy-5,8-bis[(4-hydroxybutyl)amino]-9,10-anthracenedione | |
| I-2j | 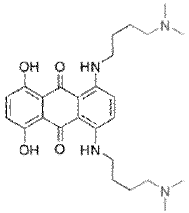 |
| NSC186848 | |
| 1,4-bis[[4-(dimethylamino)butyl]amino]-5,8-dihydroxy-9,10-anthracenedione | |
| I-2k | 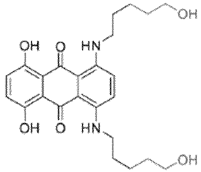 |
| NSC321458 | |
| 1,4-dihydroxy-5,8-bis(5-hydroxypentylamino)anthracene-9,10-dione | |
| I-2l | 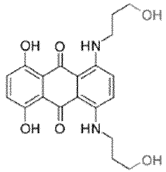 |
| NSC317017 | |
| 1,4-dihydroxy-5,8-bis[(3-hydroxypropyl)amino]-9,10-anthracenedione | |
| I-2m | 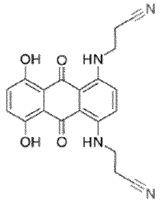 |
| NSC128432 | |
| 3-[[4-(2-cyanoethylamino)-5,8-dihydroxy-9,10-dioxoanthracen-1-yl]amino]propanenitrile | |

or a pharmaceutically acceptable salt, enantiomer, stereoisomer, or tautomer thereof.

According to a second embodiment, compounds of the present invention may thus have the general formula (IA), wherein rings A, B C, and D correspond to an anthracycline ring having the following formula:

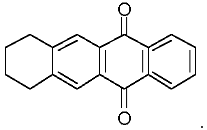

According to this second embodiment, compounds of formula IA of the present invention may be selected from the group consisting of compounds listed in the following Table 2.

**Table 2**

| | |
|---|---|
| IA-1a | 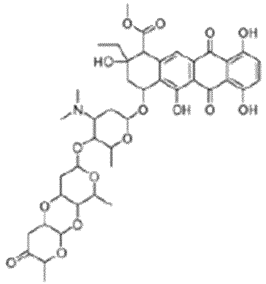 |
| NSC18334 | |
| Cinerubin A | |
| 2,3,4,6,11-hexahydro-2,5,7,10-tetrahydroxy-6,11-dioxo-4-[2,3,6-trideoxy-4-O-[2,6-dideoxy-4-O-((2R-trans)-tetrahydro-6-methyl-5-oxo-2H-pyran-2-yl)-α-L-lyxo-hexopyranosyl]-3-(dimethylamino)-α-L-lyxo-hexopyranosy-1-naphthacenecarboxylic acid | |
| IA-1b | 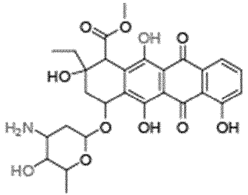 |
| NSC263854 | |
| 4-[(3-amino-2,3,6-trideoxy-α-L-lyxo-hexopyranosyl)oxy]-2-ethyl-1,2,3,4,6,11-hexahydro-2,5,7,12-tetrahydroxy-6,11-dioxo-, methyl ester, hydrochloride, [1R-(1α,2β,4β)]-1-naphthacenecarboxylic acid | |
| IA-1c | 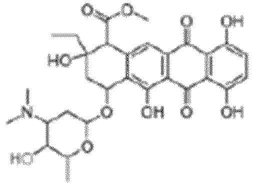 |
| NSC267229 | |
| Pyrromycin | |
| 2-ethyl-1,2,3,4,6,11-hexahydro-2,5,7,10-tetrahydroxy-6,11-dioxo-4-[[2,3,6-trideoxy-3-(dimethylamino)-α-L-lyxo-hexopyranosyl]oxy]-, methyl ester, [1R-(1α,2β,4β)]-1-naphthacenecarboxylic acid | |
| IA-1d | 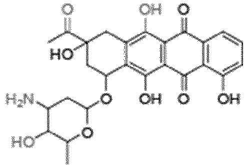 |
| NSC180024 | |
| 8-acetyl-10-[[3-amino-2,3,6-trideoxy-α-L-lyxo-hexopyranosyl]oxy]-7,8,9,10-tetrahydro-1,6,8,11-tetrahydroxy-,(8S-cis)-1-naphthacenecarboxylic acid | |
| IA-1e | 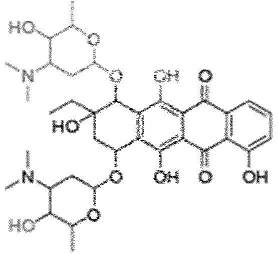 |
| NSC136044 | |
| Rhodomycin A | |
| IA-1f | 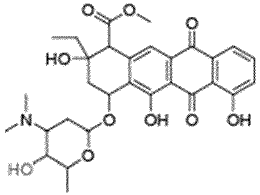 |
| NSC100290 | |
| Aklavin | |
| IA-1g | 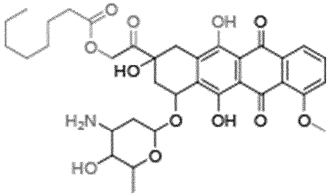 |
| NSC149584 | |
| 2-[4-[(3-amino-2,3,6-trideoxy-α-L-lyxo-hexopyranosyl)oxy]-1,2,3,4,6,11-hexahydro-2,5,12-trihydroxy-7-methoxy-6,11-dioxo-2-naphthacenyl]-2-oxoethyl ester, hydrochloride, (2S-cis)- octanoic acid | |
| IA-1h | 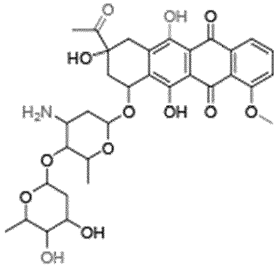 |
| NSC349631 | |
| 8-acetyl-10-[[3-amino-2,3,6-trideoxy-4-O-(2,6-dideoxy-α-L-lyxo-hexopyranosyl)-α-L-lyxo-hexopyranosyl]oxy]-7,8,9,10-tetrahydro-6,8,11-trihydroxy-1-methoxy-,(8S-cis)-5,12-naphthacenedione | |
| IA-1i | 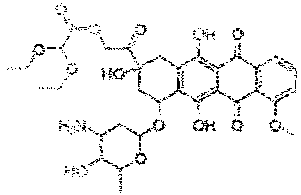 |
| NSC292652 | |
| Diethoxy-,2-[4-[(3-amino-2,3,6-trideoxy-α-L-lyxo-hexopyranosyl) oxy]-1,2,3,4,6,11-hexahydro-2,5,12-trihydroxy-7-methoxy-6,11-dioxo-2-naphthacenyl]-2-oxoethyl ester, hydrochloride, (2S-cis)- acetic acid | |
| IA-1j | 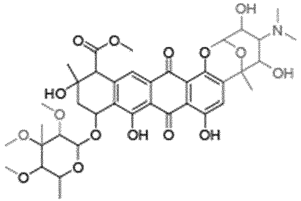 |
| NSC70845 | |
| Nogalamycin | |
| IA-1k | 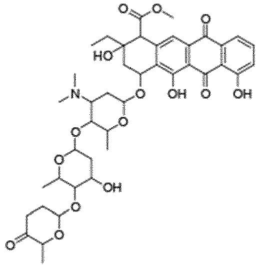 |
| NSC208734 | |
| Aclacinomycin A | |
| IA-1l | 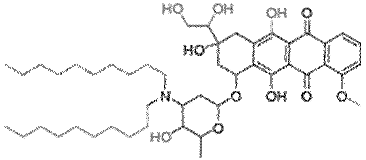 |
| NSC292686 | |
| 7-[4-(didecylamino)-5-hydroxy-6-methyloxan-2-yl]oxy-9-(1,2-dihydroxyethyl)-6,9,11-trihydroxy-4-methoxy-8,10-dihydro-7H-tetracene-5,12-dione;hydrochloride | |
| IA-1m | 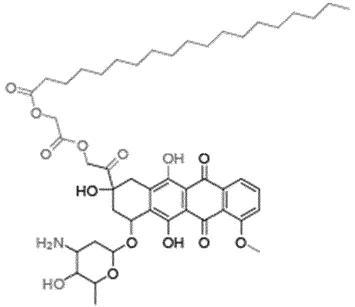 |
| NSC261057 | |
| 2-[2-[4-[(3-amino-2,3,6-trideoxy-α-L-lyxo-hexopyranosyl)oxy]-1,2,3,4,6,11-hexahydro-2,5,12-trihydroxy-7-methoxy-6,11-dioxo-2-naphthacenyl]-2-oxoethoxy]-2-oxoethyl ester, hydrochloride, (2S-cis)-octadecanoic acid | |
| IA-1n | 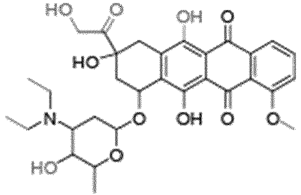 |
| NSC268239 | |
| 7,8,9,10-tetrahydro-6,8,11-trihydroxy-8-(hydroxyacetyl)-1-methoxy-10-[[2,3,6-trideoxy-3-(diethylamino)-α-L-lyxo-hexopyranosyl]oxy]-, hydrochloride, (8S-cis)- 5,12-naphthacenedione | |
| IA-1o | 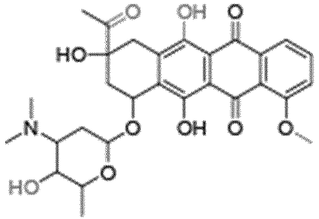 |
| NSC258812 | |
| 8-acetyl-7,8,9,10-tetrahydro-6,8,11-trihydroxy-1-methoxy-10-[[2,3,6-trideoxy-3-(dimethylamino)-α-L-lyxo-hexopyranosyl]oxy]-,hydrochloride, (8S-cis)- 5,12-naphthacenedione | |
| IA-1p | 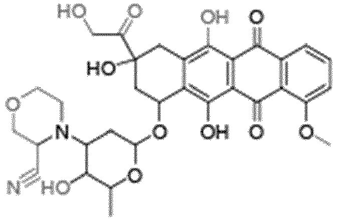 |
| NSC357704 | |
| Cyanomorpholinoadriamycin | |
| 10-[[3-(3-cyano-4-morpholinyl)-2,3,6-trideoxy-α-L-lyxo-hexopyranosyl]oxy]-7,8,9,10-tetrahydro-6,8,11-trihydroxy-8-(hydroxyacetyl)-1-methoxy-, (8S-cis)- 5,12-Naphthacenedione | |
| IA-1q | 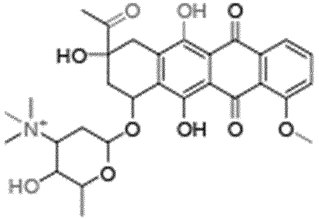 |
| NSC245426 | |
| 3-Acetyl-3,5,12-trihydroxy-10-methoxy-6,11-dioxo-1,2,3,4,6,11-hexahydrotetracen-1-yl-2,3,6-trideoxy-3-(trimethylazaniumyl)hexopyranoside chloride | |
| IA-1r | 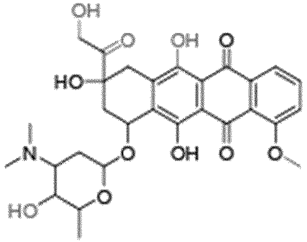 |
| NSC261045 | |
| 7,8,9,10-tetrahydro-6,8,11-trihydroxy-8-(hydroxyacetyl)-1-methoxy-10-[[2,3,6-trideoxy-3-(dimethylamino)-α-L-lyxo-hexopyranosyl] oxy]-, hydrochloride, (8S-cis)- 5,12-naphthacenedione | |

or a pharmaceutically acceptable salt, enantiomer, stereoisomer, or tautomer thereof.

According to a third embodiment, compounds of formula (II) of the present invention may have the following structure IIa or IIb: 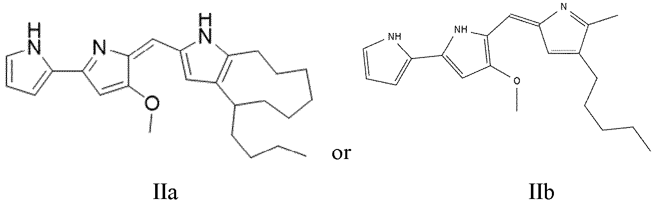 or pharmaceutically acceptable salt, enantiomer, stereoisomer, or tautomer thereof.

Compounds of formula (III) according to the fourth embodiment of the present invention may be aporphine derivatives and may be selected among compounds listed in the following Table 3.

**Table 3**

| | |
|---|---|
| IIIa | 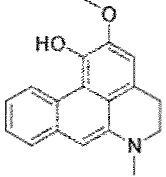 |
| NSC785168 | |
| 1-hydroxy-2-methoxydehydroaporphine hydrobromide | |
| IIIb | 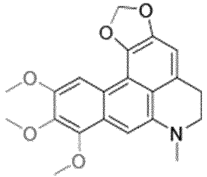 |
| NSC785155 | |
| Dehydroocopodine | |
| IIIc | 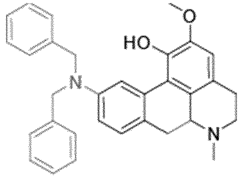 |
| NSC758176 | |
| Tranexamic acid | |
| III-3a | 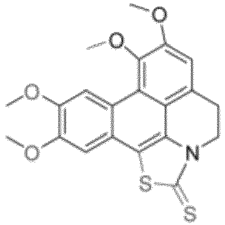 |
| NSC627604 | |
| 7,8,10,11-Tetramethoxy-4,5-dihydrodibenzo[de,g]-[1,3]thiazolo[5,4,3-ij]quinoline-2-thione | |
| III-3c | 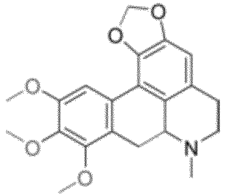 |
| NSC312326 | |
| Ocopodine (+) | |
| III-3d | 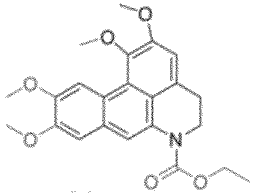 |
| NSC143241 | |
| Dibenzo[de,g]quinoline-6-carboxylic acid, 4,5-dihydro-1,2,9,10-tetramethoxy-, ethyl ester | |
| III-3e | 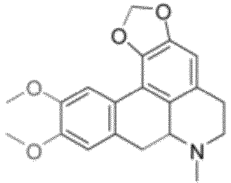 |
| NSC406035 | |
| Eximine | |
| III-3f | 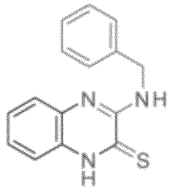 |
| NSC220254 | |
| 2-benzylamino-3-mercaptoquinoxaline | |
| or | |
| 2-benzylamino- 3-quinoxalinethiol | |
| or | |
| 3-[(phenylmethyl)amino]- 2(1H)-quinoxalinethione | |
| III-3g | 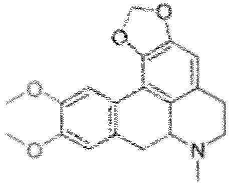 |
| NSC251699 | |
| Dicentrine (-) | |
| III-3h | 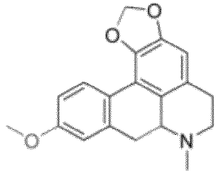 |
| NSC383229 | |
| 5H-Benzo[g]-1,3-benzodioxolo[6,5,4-de]quinoline, 6,7,7a,8-tetrahydro-10-methoxy-7-methyl- | |
| III-3i | 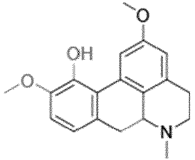 |
| NSC282458 | |
| 2,10-dimethoxy-6-methyl-5,6,6a,7-tetrahydro-4H-dibenzo[de,g] quinolin-11-ol | |
| III-3j | 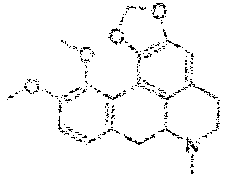 |
| NSC117866 | |
| Bulbocapnine methyl ether | |
| or | |
| N,O-dimethyl- hemangerine | |
| or | |
| 6,7,7a,8-tetrahydro-11,12-dimethoxy-7-methyl-, (S)-bulbocapnine, O-methyl- 5H-benzo[g]-1,3-benzodioxolo[6,5,4-de]quinoline | |
| III-3k | 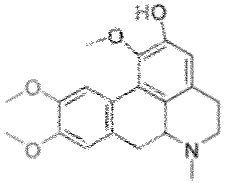 |
| NSC34396 | |
| Glaucine | |
| or | |
| 5,6,6a,7-tetrahydro-1,2,9,10-tetramethoxy-6-methyl-,(S)-6aα-aporphine, 1,2,9,10-tetramethoxy- 4H-dibenzo[de,g]quinoline | |
| III-3l | 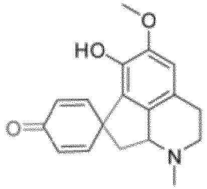 |
| NSC146052 | |
| Glaziovine | |
| 2',3',8',8'a-tetrahydro-6'-hydroxy-5'-methoxy-1'-methyl-spiro[2,5-cyclohexadiene-1,7'(1'H)-cyclopent[ij]isoquinolin]-4-one | |
| III-3m | 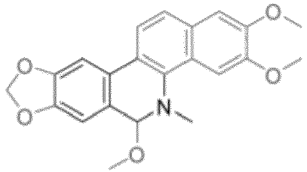 |
| NSC172620 | |
| 5,6-dihydro-2,3,6-trimethoxy-5-methyl-benzo[c][1,3]dioxolo[4,5-j] phenanthridine | |

or a pharmaceutically acceptable salt, enantiomer, stereoisomer, or tautomer thereof.

Preferred compounds according to this fourth embodiment are chosen among the following compounds: 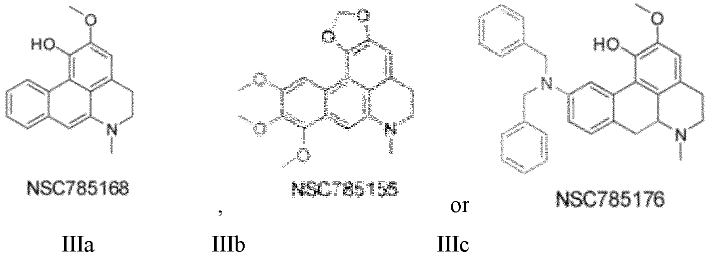 or pharmaceutically acceptable salt, enantiomer, stereoisomer, or tautomer thereof.

Compounds of the present invention are particularly useful as an anti-retroviral therapy.

The present invention also relates to a pharmaceutical composition comprising at least one compound of the present invention as described above, and a pharmaceutically acceptable carrier or excipient.

By way of example, carriers which may be used in the pharmaceutical compositions of the present invention may be saline, Cremophor EL, propylene glycol, polyethylene glycol and/or alcohol.

Pharmaceutical compositions according to the present invention may be formulated for oral, aerosol, parenteral, subcutaneous, intravenous, intraarterial, intramuscular, interperitoneal, rectal or vaginal administration.

Compounds of the present invention may be used in a method of treating and/or the preventing of a disease, disorder, or dysfunction associated with the activation of a protein kinase selected among GCN2, PKR (Protein Kinase R), HRI (Heme-regulated inhibitor), and/or PERK (PKR-like endoplasmic reticulum kinase). Preferably, compounds of the present invention may be used in a method of inhibiting GCN2 kinase or PERK kinase and/or a method of treating and/or preventing GCN2 associated disease and/or a PERK associated disease in a subject in need thereof.

As described above, they are particularly useful in a method of treating and/or preventing retroviral infection in a mammal in need thereof, comprising administering to said mammal a therapeutically effective amount of said compound or pharmaceutical composition.

Retroviral infections may be caused by retrovirus chosen among HIV-1/2, human T-cell lymphotropic virus (HTLV), feline leukemia virus, feline immunodeficiency virus, coronaviruses such as SARS-CoV-1/2, MERS-CoV or novel emerging coronaviruses, flaviviruses such as Hepatitis C virus, Zika, Dengue 1-4, Usutu, West Nile, Baiyangdian virus (BYDV) or other vector-borne orthoflaviviruses.

Human immunodeficiency virus (HIV) is a lentivirus (a member of the retrovirus family) that causes acquired immunodeficiency syndrome (AIDS). Lentiviruses are single-stranded, positive-sense, enveloped RNA viruses. Upon entry of the target cell, the viral RNA genome is converted to double-stranded DNA by a virally encoded reverse transcriptase. This viral DNA is then integrated into the cellular DNA by a virally encoded integrase (IN), along with host cellular co-factors. HIV infects primarily vital cells in the human immune system such as helper T cells (CD4+ cells), macrophages, and dendritic cells. HIV infection leads to low levels of CD4+ T cells. When CD4+ T cell numbers decline below a critical level, cell-mediated immunity is lost, and the body becomes progressively more susceptible to other viral or bacterial infections. Subjects with HIV typically develop malignancies associated with the progressive failure of the immune system. There are two main species of HIV, HIV-1 which is also termed LAV (Lymphadenopathy-Associated Virus) or HTLV-III (Human T-Lymphotropic Virus Type III) and HIV-2, which is less common (primarily found in West Africa), spreads less efficiently than HIV-1 and tends to progress more slowly to AIDS. Both species are thought to have originated from simian immunodeficiency viruses (SIVs) in primates and crossed into humans through zoonotic transmission.

Human T-Cell Leukemia Virus Type I (HTVL-I) and type II (HTLV-II) are closely related but distinct retroviruses that can infect humans. They were the first human retroviruses discovered. Both belong to the oncovirus subfamily of retroviruses and can transform human lymphocytes so that they are self-sustaining in vitro. They are only distantly related to HIV-1 and HIV-2, which belong to the lentivirus subfamily of retroviruses and which cause acquired immunodeficiency syndrome (AIDS). Infections with HTLV-I and HTLV-II are most easily detected serologically. The presence of antibodies to HTLV-I or HTLV-II indicates that a person is infected with the virus. HTLV-I is an oncogenic human retrovirus which causes a lifelong infection. Like all retroviruses, HTLV-I has two positive polarity single-stranded RNA genomes, packaged with several viral enzymes (reverse transcriptase, integrase, and protease), surrounded by capsid proteins forming a roughly spherical viral nucleocapsid. A host-derived lipid bilayer envelope, studded with viral glycoproteins (gp62, Envelope, Env) surrounds the viral capsid. As a member of the deltaretrovirus genus, HTLV-1 is a complex retrovirus that expresses several regulatory and accessory genes, in addition to the standard structural and enzymatic genes common to all retroviruses.

HTLV-II is presumed to be transmitted similarly to HTLV-I, but much less is known about the specific modes and efficiency of transmission of HTLV-II. Transmission is done by sexual contact, blood transfusion, needle sharing, and breastfeeding. The high prevalence of HTLV-II among injecting drug users is likely due to sharing blood-contaminated needles or other injection paraphernalia.

Feline leukemia virus (FeLV) is a retrovirus that infects domestic cats, resulting in significant morbidity and mortality worldwide. Though predominantly transmitted through saliva, FeLV also has been reported to spread through contact with body fluids. The clinical signs in cats observed during FeLV infections include cyto-proliferative disorders (lymphoid or myeloid tumors), cyto-suppressive disorders (infectious diseases associated with immunosuppression, anaemia, myelosuppression), inflammatory disorders, neurological disorders, abortions, and enteritis. The RNA genome of FeLV comprises the ENV (Envelope) gene, which encodes the proteins involved in the virus's entry into host cells, such surface protein gp70, transmembrane protein p15E, (ii) a GAG (Group-Specific Antigen) gene, which encodes structural components that forms the viral core and the matrix of the virus, *i.e.,* the matrix protein p15, the capsid protein p27 and the nucleocapsid protein p10, (iii) a POL (Polymerase) gene, which encodes the reverse transcriptase, the integrase, and the protease.

Feline immunodeficiency virus (FIV), formerly called feline T-lymphotropic lentivirus, was first isolated in 1986 from a large multiple-cat household in Petaluma, California. FIV infects cats and produces an AIDS-like syndrome. Although FIV is morphologically and pathologically similar to human immunodeficiency virus (HIV), it has been shown to be antigenically distinct from HIV. Like HIV, once a cat becomes infected with FIV, the disease progresses through several stages: (1) primary infection: Characterized by viremia, fever, and generalized lymphadenitis; (2) asymptomatic phase: A prolonged period where the infected cat shows no apparent clinical signs, and (3) immunodeficiency phase: Severe impairment of immune function caused by a reduction in CD4+ lymphocytes, leading to increased susceptibility to secondary infections and, ultimately, death. FIV has been classified as a member of the subfamily Lentivirinae within the family Retroviridae, which also includes human and simian immunodeficiency viruses, equine infectious anemia virus, Maedi-Visna virus (MVV) of sheep, and caprine arthritis encephalitis virus (CAEV). The FIV genome is organized like other lentiviruses, with three major long open reading frames corresponding to GAG, POL, and ENV. The GAG gene encodes the major structural components of the virus. The ENV gene encodes the envelope glycoproteins. The POL gene encodes the polymerase protein. The GAG gene is expressed as a 55 kDa polyprotein, which is processed into three subunits: a p15 matrix protein, a p24 capsid protein, and a p10 nucleocapsid protein. The POL gene encodes three key proteins: a protease, a reverse transcriptase, an integrase which facilitates the integration of viral DNA into the host cell's genome, and a p14.6 protein of unknown function. Autoprocessing by the protease portion of the POL gene produces all three proteins in this region. Additionally, the protease is responsible for processing the gag precursor. The pol gene is expressed as a gag-pol fusion protein. The ENV gene is expressed as a 160 kDa glycoprotein (gp160), which is cleaved into two subunits: gp120, the surface glycoprotein, and gp41, the transmembrane glycoprotein. Coronaviruses are enveloped, positive-sense single-stranded RNA viruses. A key feature of these viruses is the large, trimeric spike (S) glycoprotein on their surface, which plays a crucial role in the infection process by mediating both binding to host cell receptors and the fusion of viral and host cell membranes. The S protein is composed of two functional subunits: S1 subunit: Responsible for receptor binding and the S2 subunit: Responsible for membrane fusion. Recent cryo-electron microscopy (cryo-EM) reconstructions of the trimeric S protein structures from alpha-, beta-, and deltacoronaviruses have provided detailed insights into the architecture of the S1 subunit. It is organized into two distinct domains: N-terminal domain (S 1 NTD) and Receptor-binding domain (S1 RBD). In the case of SARS-CoV-2, the S1 RBD specifically binds to the human angiotensin-converting enzyme 2 (ACE2) receptor, initiating the viral entry process. This interaction is a critical determinant of SARS-CoV-2's infectivity and host specificity, and it represents a key target for therapeutic interventions, such as vaccines and neutralizing antibodies.

Coronaviruses are known to cause severe respiratory and gastrointestinal diseases in animals. In humans, coronavirus infections have long been associated with respiratory tract diseases, ranging from mild common cold-like symptoms to severe respiratory conditions. For example, SARS-CoV (Severe Acute Respiratory Syndrome Coronavirus) is a highly pathogenic human virus that causes acute respiratory distress syndrome (ARDS), often with fatal outcomes. SARS-CoV emerged as an epidemic in 2003 after crossing the species barrier from bats to civet cats and humans. This outbreak highlighted the potential of coronaviruses to cause high morbidity and mortality in humans.

Following this, two additional human coronavirus strains, HCoV-NL63 and HCoV-HKU1, were discovered in 2004 and 2005, respectively. In 2012, another highly pathogenic human coronavirus, MERS-CoV (Middle East Respiratory Syndrome Coronavirus, previously referred to as "EMC"), emerged in the Middle East. MERS-CoV caused clinical outcomes such as acute pneumonia and renal failure, with a mortality rate of approximately 50%, surpassing that of SARS-CoV.

In late 2019, Severe Acute Respiratory Syndrome Coronavirus 2 (SARS-CoV-2) was identified as the causative agent of COVID-19, a disease that rapidly escalated into a global pandemic. The World Health Organization (WHO) has declared COVID-19 a "global public health threat." In most cases, COVID-19 causes mild symptoms. However, in 16-20% of patients, the disease can progress to severe forms requiring oxygen support and hospitalization. Approximately 5-10% of cases develop acute respiratory distress syndrome (ARDS), necessitating admission to intensive care units (ICUs) and invasive mechanical ventilation. ARDS is responsible for 99% of ICU admissions and COVID-19-related deaths. ARDS in COVID-19 is characterized by respiratory failure resulting from impaired gas exchange due to alveolar-capillary barrier edema. This lung injury is driven by a massive, dysregulated inflammatory response to viral invasion of lung epithelial cells, leading to a cascade of immune-mediated damage.

The long duration of ICU stays in severe COVID-19 cases places significant strain on healthcare systems, further emphasizing the need for effective prevention and management strategies.

Coronaviruses belong to the family Coronaviridae and are classified into four genera: Alphacoronavirus, Betacoronavirus, Deltacoronavirus, and Gammacoronavirus. There are also unclassified coronaviruses within this family. Human-infecting coronaviruses primarily belong to the Alphacoronavirus and Betacoronavirus genera. Notable examples include: SARS-CoV (2003 epidemic), MERS-CoV (2012 outbreak), SARS-CoV-2 (2019 pandemic, previously called "Wuhan Human Coronavirus" or "nCoV-2019"). Coronaviruses are genetically highly variable, a feature that allows them to adapt to different hosts and cross species barriers. This genetic flexibility has been a significant factor in the emergence of new human-infecting coronaviruses, as seen with SARS-CoV, MERS-CoV, and SARS-CoV-2.

Hepatitis C Virus (HCV) is an enveloped, positive-sense single-stranded RNA virus belonging to the Flaviviridae family. Its genome is approximately 9,500 nucleotides in length and contains a single open reading frame (ORF), which encodes a large polyprotein of about 3,000 amino acids. In infected cells, this polyprotein is cleaved at multiple sites by cellular and viral proteases to generate both structural and non-structural (NS) proteins essential for viral replication and assembly. The cleavage of the HCV polyprotein into functional proteins is mediated by both cellular proteases and viral proteases: structural proteins which form the viral envelope and capsid; and non-structural (NS) proteins: NS2, NS3, NS4A, NS4B, NS5A, and NS5B, which are critical for viral replication. The generation of mature non-structural proteins is facilitated by two viral proteases: NS2 protease which cleaves specifically at the NS2-NS3 junction of the polyprotein; and NS3 protease, which is a serine protease located in the N-terminal region of NS3, which mediates all subsequent cleavages downstream of NS3 in the polyprotein. These cleavage sites include NS3-NS4A, NS4A-NS4B, NS4B-NS5A, NS5A-NS5B. The NS3 protease exhibits cleavage activity in both cis (at the NS3-NS4Ajunction) and trans (at downstream sites). This activity is enhanced by NS4A, a cofactor protein that: facilitates the formation of the NS3-NS4A complex, which is essential for efficient proteolytic processing, and may assist in membrane localization of NS3 and other viral replication components. In addition to its protease function, NS3 also exhibits nucleoside triphosphatase (NTPase) activity and an RNA helicase activity, which is crucial for unwinding RNA structures during replication. The NS5B is an RNA-dependent RNA polymerase (RdRp) that plays a central role in replicating the viral RNA genome. It synthesizes new RNA strands using the viral RNA as a template.

HCV is a major human pathogen, infecting an estimated 170 million people worldwide, a figure roughly five times greater than those infected with HIV-1. Chronic HCV infection leads to serious and progressive liver diseases, including cirrhosis and hepatocellular carcinoma (HCC). HCV represents a significant global health challenge due to its high prevalence and severe long-term consequences. Its replication and pathogenesis are driven by the intricate processing and function of its polyprotein, particularly the NS3 protease, which serves as a critical target for direct-acting antivirals (DAAs).

Zika virus is an arbovirus belonging to the Flaviviridae family. It is a member of the Spondweni serocomplex and is closely related to other flaviviruses, including yellow fever virus, dengue virus, West Nile virus, and Japanese encephalitis virus. Like other members of the Flavivirus genus, Zika virus contains a positive-sense, single-stranded RNA genome that encodes a polyprotein, which is cleaved into several structural and non-structural proteins. Virus replication occurs within the cytoplasm of host cells.

Zika virus was first isolated in 1947 from a sentinel rhesus monkey placed in the Zika Forest of Uganda. For many years, Zika virus attracted little global attention, as it was confined to a narrow equatorial belt across Africa and Asia. However, after large outbreaks in French Polynesia in 2013 and in Brazil, Colombia, and Cape Verde in 2015, Zika virus emerged as a significant global public health concern. The explosive pandemic in much of South and Central America prompted the World Health Organization (WHO) to declare the Zika virus outbreak a 'Public Health Emergency of International Concern' in 2016.

In humans, Zika virus infection typically causes a dengue-like illness with symptoms such as: fever, muscle aches, eye pain, prostration, and maculopapular rash. Unlike dengue fever, no cases of hemorrhagic fever have been reported in Zika virus infections.

Zika virus has transitioned from a neglected tropical disease to a major emerging pathogen, largely due to its ability to spread rapidly and its association with severe health outcomes, including congenital and neurological disorders. Efforts to combat Zika virus include public health measures to control mosquito populations (e.g., Aedes aegypti, the primary vector), development of vaccines, and raising awareness about the risks of infection during pregnancy.

The dengue viruses (DENV) belong to the genus Flavivirus in the family Flaviviridae. This genus includes other significant human pathogens transmitted by mosquitoes and ticks, such as yellow fever virus, West Nile virus, Japanese encephalitis virus, and tick-borne encephalitis virus. Dengue infections are caused by four closely related serotypes: DENV-1, DENV-2, DENV-3, and DENV-4. These serotypes share approximately 65% of their genome but differ enough in their antigenic properties to elicit unique interactions with the human immune system. Although genetic variations exist within each serotype, infection with any of the four serotypes can result in similar clinical presentations, ranging from mild fever to severe dengue.

DENV-1 is the first discovered and widely reported serotype. It causes classic dengue symptoms such as high fever, muscle pain, and rash. The infection with DENV-1 may decrease immunity to other serotypes, increasing the risk of severe dengue in subsequent infections due to a phenomenon called antibody-dependent enhancement (ADE). DENV-2 is known for causing more serious manifestations, including severe dengue and Dengue Shock Syndrome (DSS). It is associated with higher rates of bleeding, dangerous drops in blood pressure, and severe complications.

It is frequently implicated in large outbreaks in endemic areas. DENV-3 can cause symptoms like other serotypes but is often associated with immune enhancement phenomena, increasing the risk of severe symptoms upon subsequent exposure to a different serotype. It plays a role in severe dengue cases in regions where it co-circulates with other serotypes. DENV-4 is the most recently identified serotype. It is less commonly recognized but still capable of causing serious infections. Early exposure to DENV-4 can increase susceptibility to severe disease upon infection with other serotypes in the future.

The dengue virus genome consists of a single strand of positive-sense RNA, approximately 10.7 kilobases (kb) in length. This genome can be directly translated into proteins by the host cell machinery. It encodes a single, long polyprotein, which is cleaved into ten functional proteins: three structural proteins: the capsid (C) which forms the viral nucleocapsid, the Envelope € which mediates host cell attachment and entry, and the membrane (M): Plays a role in viral assembly and maturation. Seven non-structural proteins: NS1 (essential for viral replication and immune evasion); NS2A (which plays a role in replication complex formation; NS2B (a cofactor for the viral protease (NS3); NS3 (which functions as a protease and helicase, critical for viral replication), NS4A (involved in modifying host cell membranes to support replication); NS4B (which contributes to viral replication and immune evasion); NS5 (which functions as an RNA-dependent RNA polymerase (RdRp) and a methyltransferase, essential for RNA synthesis and capping).

Dengue virus infections range from asymptomatic cases to severe dengue, which can include Dengue Hemorrhagic Fever (DHF) and Dengue Shock Syndrome (DSS). A key challenge in dengue management is antibody-dependent enhancement (ADE), where preexisting antibodies from a previous infection with one serotype can enhance the severity of a subsequent infection with a different serotype.

Dengue is a major public health concern, particularly in tropical and subtropical regions. With millions of cases annually, dengue virus remains one of the most important arboviral pathogens worldwide. Its ability to co-circulate and evolve among multiple serotypes poses significant challenges for vaccine development and epidemic control.

Usutu Virus (USUV) is an emerging arbovirus that was first isolated in South Africa in 1959. This Flavivirus is maintained in the environment through an enzootic cycle involving mosquitoes as vectors and birds as reservoir hosts. Over the past two decades, USUV has spread across much of the European continent, causing significant avian mortality. In recent years, a recrudescence of bird infections has been recorded throughout Europe.

In humans, USUV infection is most often asymptomatic or causes mild clinical signs. However, there have been a few reported cases of neurological complications, including encephalitis and meningoencephalitis. While USUV shares many features with West Nile virus (WNV), including a close phylogenetic relationship and a similar ecology, USUV has been less extensively studied. Co-circulation of USUV and WNV is frequently observed in nature.

USUV is an enveloped virus approximately 40-60 nm in diameter, with a single-stranded RNA genome of positive polarity. The genome is 11,064 nucleotides in length, capped at the 5' end with an N7-methylguanosine-triphosphate cap, but it lacks a polyA tail at the 3' end. The genome encodes a single open reading frame (ORF), which is translated into a polyprotein of 3,434 amino acids. This polyprotein is cleaved into: three structural proteins: Capsid (C), Premembrane (prM), and Envelope (E) and eight non-structural proteins: NS1/NS1', NS2A, NS2B, NS3, NS4A, 2K, NS4B, and NS5.

Structural proteins include capsid (C) which forms the central body of the virion and associates with the viral RNA; premembrane (prM) which facilitates virion assembly and maturation by stabilizing and folding the envelope glycoprotein (E); envelope (E) which plays critical roles in viral attachment to host cells and fusion with the cell membrane, enabling viral entry.

Non-structural proteins are primarily associated with the endoplasmic reticulum (ER), where they form replication complexes. NS5 is the largest and most conserved protein, performs RNA-dependent RNA polymerase (RdRp) activity for viral RNA replication. It also contains a methyltransferase domain responsible for capping the viral RNA at the 5' end, ensuring RNA stability and efficient translation. Similar to other flaviviruses, USUV replicates in the cytoplasm of infected cells. Non-structural proteins interact with the ER to create a replication complex, where RNA synthesis occurs.

The level of genetic relatedness among USUV strains is influenced by their geographical origin and the host species from which they were isolated. Comparative genomic analyses have identified specific amino acid mutations associated with geographical and host variations, which may influence viral replication, host adaptation, or pathogenicity. Although USUV was initially of limited interest due to its restricted distribution and mild human impact, its rapid geographic expansion and association with avian die-offs in Europe, as well as neurological complications in humans, highlight its potential as a public health threat. More comprehensive studies are required to better understand the biology, pathogenesis, and ecology of USUV and its interaction with other co-circulating arboviruses, such as WNV.

West Nile Virus (WNV) is a mosquito-borne virus that primarily infects birds, which serve as the main reservoirs of the virus. However, humans and mammals can also become infected. In humans, approximately 80% of infections are asymptomatic, while the remaining cases may range from mild febrile illness to severe neurological disease. Most human infections occur between July and September, coinciding with peak mosquito activity. WNV was first recognized in North America in 1999 during an outbreak in New York City, and it has since rapidly established itself across the continent. Historically, WNV was associated with sporadic outbreaks of mild febrile illness. However, recent years have seen a shift in its epidemiology and clinical features, with more frequent outbreaks and a higher proportion of cases involving severe disease.

WNV is transmitted to birds and other hosts through mosquito bites, primarily by species of the genus Culex. Although humans and mammals are considered dead-end hosts (as they do not produce sufficient viremia to infect mosquitoes), WNV can infect a wide range of species, including birds (primary reservoir hosts), mosquitoes (vectors), horses, and other mammals.

While most human infections are asymptomatic, symptomatic cases may present in two main forms: (1) West Nile Fever, a mild, self-limiting illness characterized by fever, headache, muscle aches, and fatigue; or (2) neuroinvasive Disease: a severe condition affecting the central nervous system, which can lead to encephalitis, meningitis, or acute flaccid paralysis. Neuroinvasive disease is more common in older adults, immuno-compromised individuals, or those with pre-existing health conditions. Severe cases can result in long-term neurological complications or death.

WNV is a member of the Flavivirus genus in the family Flaviviridae and belongs to the Japanese encephalitis antigenic complex. Its positive-sense single-stranded RNA genome is approximately 11 kb in length. The genome encodes 10 proteins as a single polyprotein, flanked by highly structured 5' and 3' untranslated regions (UTRs), which play crucial roles in the virus's lifecycle.

Key Genomic Features include (1) 5' UTR which contains a conserved RNA structure known as Stem Loop A (SLA), which functions as a promoter for genome replication by recruiting the viral polymerase NS5; and (2) 3' UTR, one of the most well-studied regions of the WNV genome, it facilitates the immune evasion by producing subgenomic flaviviral RNA (sfRNA), which interferes with host antiviral responses and the pathogenesis, as sfRNA contributes to viral virulence. Despite detailed studies on sfRNA subdomains, the impact of the entire 3' UTR structure on sfRNA folding and production remains incompletely understood. Nonstructural proteins include NS5, the viral RNA-dependent RNA polymerase (RdRp), which plays a critical role in RNA synthesis and capping of the viral genome.

WNV continues to pose a significant public health threat, particularly in temperate regions where mosquitoes are active during the summer months. Efforts to control WNV focus on mosquito control programs, including insecticide use and reduction of standing water; public awareness campaigns to prevent mosquito bites (e.g., use of repellents, protective clothing, and window screens), and surveillance programs to monitor WNV activity in birds, mosquitoes, and humans.

While WNV was initially associated with mild illnesses, its ability to cause neuroinvasive disease and outbreaks underscores the need for continued research and monitoring. Advances in understanding the structure and function of the viral genome, particularly the UTRs and sfRNA, may lead to novel therapeutic approaches and better strategies to combat WNV infections.

A new emerging duck egg drop syndrome (DEDS) epidemic has recently spread across duck-production provinces in several countries, causing significant economic losses to the poultry industry. Infected ducks display clinical symptoms, including anorexia, diarrhea, ataxia, paralysis, and most notably, a severe decline in egg production. This dramatic drop in egg yield is the hallmark of the disease. The causative agent of this epidemic is a newly identified flavivirus named duck egg drop syndrome virus (DEDSV), also known as Baiyangdian virus (BYD virus). Genetically, DEDSV is closely related to Tembusu virus (TMUV) and Sitiawan virus (STWV). Despite this close genetic relationship, DEDSV differs in its disease association and host spectrum. Although some literature refers to DEDSV as duck TMUV, it is distinct from other Tembusu-related viruses.

In addition to infecting adult laying ducks, DEDSV has been isolated from young meat-type ducks, geese, house sparrows, and dead racing pigeons. This broad host range raises concerns about the potential for DEDSV to affect other avian species.

Like other flaviviruses, mature DEDSV virions consist of a nucleocapsid, which encases the viral RNA; a phospholipid bilayer envelope, which surrounds the nucleocapsid. Two viral structural proteins embedded in the surface of the virion include the envelope protein (E) which facilitates viral attachment and entry into host cells and the membrane protein (M) which plays a role in viral assembly and maturation.

The DEDSV genome is 10,990 nucleotides (nt) long and has the following features a type 1 cap structure (m7GpppAmp) at the 5' end, which ensures RNA stability and translation; the genome lacks a poly(A) tail at the 3' end, the open reading frame (ORF), flanked by 5' and 3' non-coding regions (NCRs) of 94 nt and 618 nt, respectively, encodes a large polyprotein of 3,425 amino acids.

The polyprotein undergoes co- and post-translational cleavage by the host signalase, which processes structural proteins, and a viral serine protease, which processes nonstructural proteins. This cleavage produces 10 viral proteins, which is typical of flaviviruses: three structural proteins including the capsid (C) which encases the viral RNA, the premembrane (PrM) which assists in viral assembly and maturation, the envelope (E) which is critical for host cell attachment and membrane fusion. Seven nonstructural proteins including NS1 (having a role in immune evasion and viral replication); NS2A and NS2B (involved in replication complex formation); NS3: (which functions as a protease and helicase, essential for RNA replication); NS4A and NS4B: (which modify host cell membranes to support replication); and NS5 (which contains RNA-dependent RNA polymerase (RdRp) and methyltransferase activities, crucial for RNA synthesis and 5' capping).

DEDSV is a significant threat to the poultry industry due to its ability to cause severe economic losses and its potential to spread among various bird species. Ongoing research focuses on genomic characterization of the virus, understanding its pathogenesis and host adaptation mechanisms, and exploring potential strategies for disease prevention and control.

Orthoflavivirus is a genus of positive-sense single-stranded RNA (ssRNA+) viruses in the Flaviviridae family. These viruses infect vertebrates and are transmitted by arthropod vectors, classifying them as arboviruses. In humans, orthoflaviviruses are associated with febrile illnesses that can present with variable symptoms, ranging from mild fever to severe neurological complications. Orthoflaviviruses are enveloped and spherical, with a diameter of approximately 50 nm. The surface proteins are arranged in a structure resembling icosahedral symmetry. Mature virions contain two membrane proteins: the membrane (M) protein and the nvelope (E) protein, which mediates attachment and fusion with host cell membranes. Immature virions contain a precursor membrane (prM) protein, which is cleaved to M during the maturation process.

The genome of orthoflaviviruses is a single positive-sense RNA strand, flanked by 5' and 3' untranslated regions (UTRs). At the 5' end, the genome contains a methylated nucleotide cap, which facilitates canonical cellular translation by host ribosomes. At the 3' end, the genome lacks a poly(A) tail but forms a secondary loop structure, which plays critical roles in viral replication and host immune evasion.

Orthoflaviviruses are of significant medical importance due to their association with various human diseases. These viruses leverage sophisticated interactions between their genome structures and the host's cellular machinery to promote viral replication, immune evasion, and pathogenesis.

Compounds of Formula I, IA, II and III as defined herein, may be used in combination with one or more additional therapeutic agents to treat a disorder described herein, such as one or more anti-viral or anti-retroviral agent.

By way of examples, said one or more anti-viral or anti-retroviral agent that may be chosen among protease inhibitors, including, but not limited to, saquinavir, indinavir, ritonavir, nelfinavir, atazanavir, darunavir, fosamprenavir, tipranavir and amprenavir; nucleoside reverse transcriptase inhibitors including but not limited to, zidovudine, didanosine, stavudine, lamivudine, zalcitabine, emtricitabine, tenofovir disoproxil fumarate, AVX754 and abacavir; non-nucleoside reverse transcriptase inhibitors including, but not limited to, nevaripine, delavirdine, calanolide A, TMC125 and efavirenz; combination drugs including, but not limited to, efavirenz/emtricitabine/tenofovir disoproxil fumarate, lamivudine/zidovudine, abacavir/lamivudine, abacavir/lamivudine/zidovudine, emtricitabine/tenofovir disoproxil fumarate, sulfamethoxazole/trimethoprim, and lopinavir/ritonavir; entry and fusion inhibitors, including, but not limited to, enfuvirtide, AMD070, BMS-488043, fozivudine tidoxil, GSK-873,140, PRO 140, PRO 542, Peptide T, SCH-D, TNX-355, and UK-427,857; treatments for opportunistic infections and other conditions associated with AIDS and HIV including, but not limited to, acyclovir, adefovir dipivoxil, aldesleukin, amphotericin b, azithromycin, calcium hydroxylapatite, clarithromycin, doxorubicin, dronabinol, entecavir, epoetin alfa, etoposide, fluconazole, ganciclovir, immunoglobulins, interferon alfa-2, ionomycine, isoniazid, itraconazole, megestrol, paclitaxel, peginterferon alfa-2, pentamidine, poly- 1 -lactic acid, ribavirin, rifabutin, rifampin, somatropin, testosterone, trimetrexate, and valganciclovir; integrase inhibitors including, but not limited to, GS 9137, MK-0518; microbicides, including, but not limited to, BMS-378806, C31G, carbopol 974P, carrageenan, cellulose sulfate, cyanovirin-N, dextran sulfate, hydroxyethyl cellulose, PRO 2000, SPL7013, tenofovir, and UC-781, and IL- 2.

Additional therapeutic agents may comprise hormonal therapeutic agents, immunotherapeutic agents, medicaments inhibiting actions of cell growth factor and receptor thereof, inhibitors of PKR (Protein Kinase R), HRI (Heme-regulated inhibitor), PERK (PKR-like endoplasmic reticulum kinase), autophagy inhibitors, the enzyme asparaginase (ASNase), and the like.

In an embodiment, the compounds of the present invention provide a wide range of activity against the IN-GCN2 interaction. Thus, starting from cinerubin A, cinerubin B and NSC263854 (1b) that were the most active compounds in this study, it appears possible to develop an inhibitor that has better affinity for the IN-GCN2 interaction without the drawbacks of canonical anthracycline derivatives.

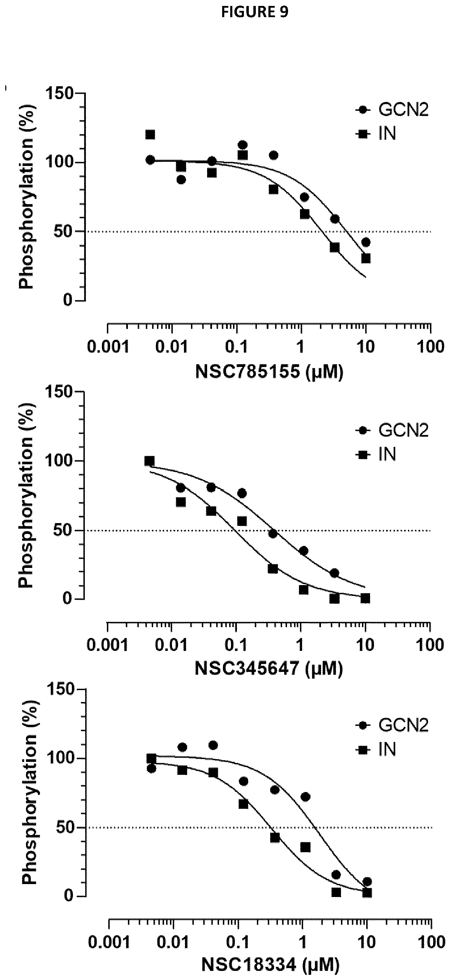

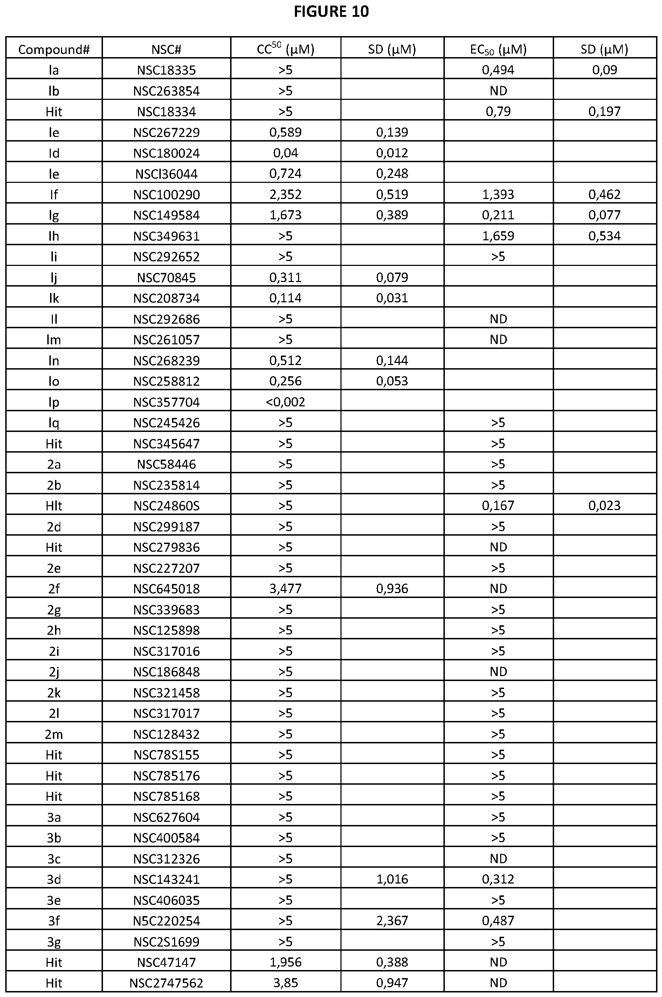

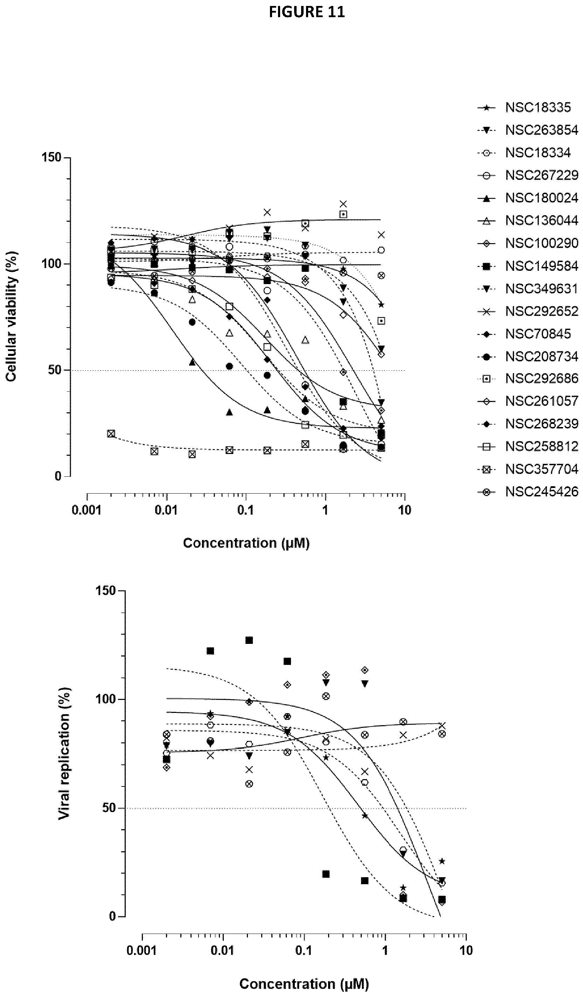

Finally, a number of these compounds were tested in cells for their antiviral and cytotoxic activity (Figures 9-11). Compounds not showing toxicity up to 5µM were tested for their antiviral activity. The most active molecules (Figure 10) act at low concentrations, below the micromolar mark with the best to a few hundred nanomolars. The most interesting compounds, NSC248605, NSC18335 (1a), NSC149584 (1g) and NSC18334 have a selectivity index ranging from 6 to more than 30. New syntheses in larger quantities would be necessary to determine the exact toxicity compounds.

### EXAMPLES

### Example 1: Material and Methods

### Chemicals

The Approved Oncology Drugs Set VIII (133 molecules), the Natural Products Set V (420 molecules) and individual molecules used in this study were obtained through the National Cancer Institute Developmental Therapeutics Program (NCI DTP, NIH). Molecules were provided either as 10 mM stock solutions in 100% dimethylsulfoxide (DMSO) or as powder that were dissolved the same way and stored at -20°C.

### Proteins

GCN2 was commercially available from SignalChem (EIF2AK4 (GCN2), cat. #E12-11G). It is an active truncated version of the human protein (192-1024) fused with a N-terminal glutathione S-transferase (GST) tag for a molecular weight of about 132 kDa.

Stock solutions were provided at a concentration of 0.1 mg/ml in storage buffer [50 mM Tris-HCl pH 7.5; 150 mM NaCl; 10 mM glutathione; 0.1 mM EDTA; 0.25 mM dithiothreitol (DTT); 0.1 mM phenylmethanesulfonyl (PMSF); 25% glycerol] and stored at -80°C.

Recombinant full-length HIV-1 IN was produced in-house as previously described (Metifiot, M. et al., Biochemistry 2010, 49, 3715-3722, doi:10.1021/bi100130f). Briefly, expression of IN was obtained by transformation of BL21(DE3)pLysS (Invitrogen) with a pET15b vector (N-terminal 6xHis tag) and induction by isopropyl β-D-1-thiogalactopyranoside (IPTG). The resulting 6xHis-IN fusion protein was purified using
Ni-NTA agarose (Qiagen) loaded on Poly-prep chromatography column (Bio-Rad). Elution was performed with increasing imidazole concentrations (20, 60, 100, 250, 750 mM). Protein purity in each fraction was observed on a 10% denaturing SDS-PAGE and Coomassie Brilliant Blue R-250 staining. Fractions of interest were then dialyzed (Thermo Scientific, Slide-A-Lyzer 10K MWCO) to remove imidazole (25 mM PIPES pH 6.8; 750 mM
NaCl; 50 µM ZnCl2;.0.1 mM EDTA; 50% glycerol). Protein concentration was measured by spectrophotometry (Thermo Scientific, Nanodrop 2000) (Molecular weight: 34 kDa, extinction coefficient: 61420 L.mol-1.cm-1) and solutions were stored at -20°C.

### IN-GCN2 Interaction Assay

The interaction between IN and GCN2 was monitored using the AlphaLISA technology (Revvity). The reaction was performed in a shallow-well 384-well microplate (Revvity, AlphaPlate-384SW, ProxiPlate) with 24 nM GST-GCN2, 700 nM 6xHis-IN, 10 µg/mL of nickel chelate acceptor beads and glutathione donor beads, the test compound or an equivalent amount of DMSO (solvent at 10 % final concentration) in a buffer composed of 50 mM Tris-HCl pH 7.5, 1 mM DTT, 0.05% Tween 20, 0.1% bovine serum albumin (BSA) and 40 mM NaCl (final volume 15µl). First, the reaction was initiated by adding the various components except the donor beads. After 20 minutes at room temperature, the donor beads were added and the plate sealed. Data acquisition was performed 120 minutes later using a Victor Nivo Multimode Plate Reader (Revvity). Means, standard deviations and graphical representation were generated using Prism 8.4.3 software (GraphPad).

### Phosphorylation assay

*In vitro* phosphorylation assay was performed by incubating the kinase GST-GCN2 (24 nM ) with its substrate His-IN (700 nM) in a reaction buffer composed of 15 mM MgCl2, 10 mM DTT, 10 mM Tris-HCl pH 8.0, 100 µM ATP, 7.4 mM MnCl2, 0.05% NP40, 1.5 µCi ATP [γ-32P] 6000 Ci/mmol. Reaction mix was incubated at 30°C for 60 minutes and stopped by the addition of Laemmli buffer (50 mM Tris pH 6.8, 2% SDS, 10% Glycerol, 5% β-mercaptoethanol, 0.2% bromophenol blue). Samples were separated on a 10% denaturing SDS-PAGE and proteins were stained using Coomassie Brilliant Blue R-250. Phosphorylation was revealed by autoradiography (Fujifilm, Imaging Plate BAS-MS) after overnight exposure and imaging on a FLA-5000 Imaging System (Fujifilm). Phosphorylation signals were quantified and analyzed using ImageQuant TL 10.1 analysis software
(Cytiva) and Prism 8.4.3 software (GraphPad).

### Example 2: Screen of natural products on the IN-GCN2 interaction

420 molecules from the National Cancer Institute Developmental Therapeutics Program (NCI DTP) were screened as modulators of the IN-GCN2 interaction *in vitro.* Each molecule was tested at a single dose (100 µM final concentration) and compared to the DMSO control (Figure 1A). Experiments were conducted onto two separate AlphaPlates 384-shallow well, with 180 and 240 molecules respectively and 12 DMSO control each. The average signal obtained with DMSO was 3905 +/- 421 and 5608 +/-856, which were considered as the 100% values for the corresponding plate (Figure 1A). Based on the highest standard deviation of the 2 plates (15%), the usual 3xSD threshold was not stringent enough and too many molecules would have been selected for validation (74 inhibitors below 55% residual interaction and 9 stimulators above 45% increase in signal). Thus, a threshold was selected at 10% residual signal (corresponding to 90% inhibition of the interaction, 9 molecules) and at 200% (a 165 2-fold increase in signal, 2 molecules).

Accordingly, only two compounds were considered as stimulators, NSC76022 and NSC267033, increasing the signal to 265.2% and 218.5% compared to DMSO, respectively.

Regarding the inhibitors, NSC785168 was the most effective with more than 95% decrease in signal (4.5% residual signal compared to DMSO). NSC18334, NSC47147, NSC345647, NSC248605, NSC785165, NSC785155, NSC247562, NSC785176 inhibited the IN-GCN2 interaction with only 5.5 to 9% residual signal compared to the DMSO control. Of note, 2 out of 9 inhibitors (NSC18334 and NSC345647) and 1 out of 2 stimulators (NSC76022) were on the first screen plate containing 180 test compounds.

### Example 3: Chemotype identification and Selection of derivatives

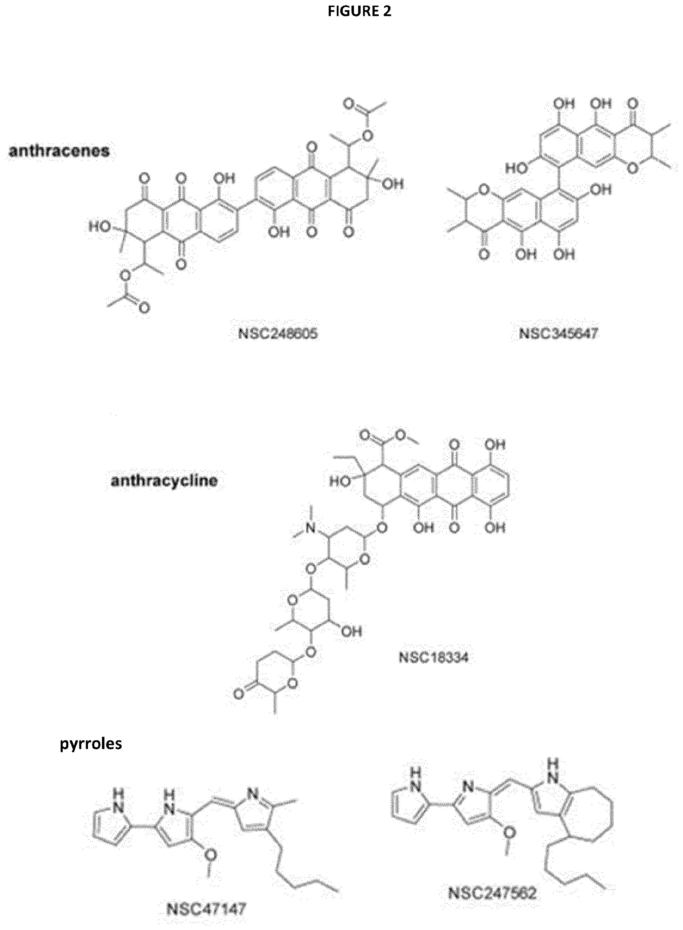

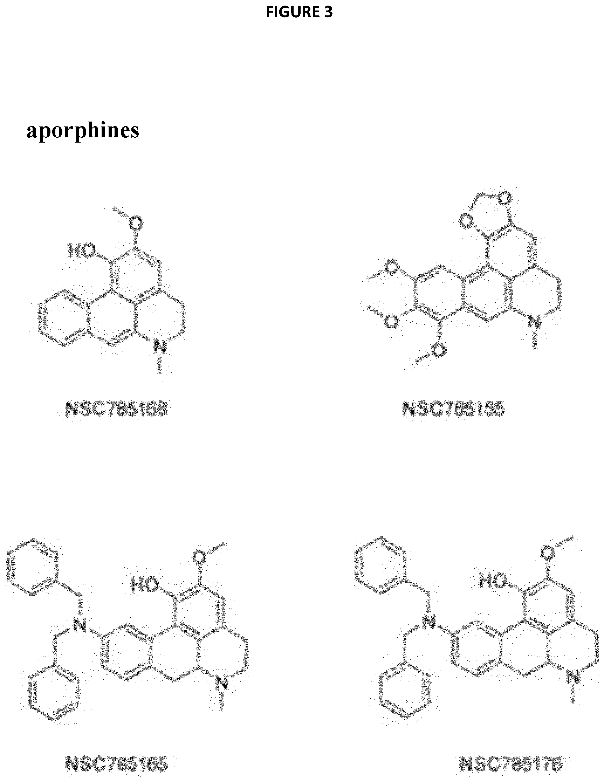

On a chemical point of view, the 2-dimensional (2D) structure of the nine molecules selected is presented in Figures 2 and 3. The most active compound, NSC18334, is a tetracycline with a side chain composed of a succession of 3 tetrahydropyran groups (THP).

NSC248605 and NSC345647 are bianthracene derivatives, with NSC248605 presenting a comparable anthraquinone arrangement than that observed in NSC18334. Interestingly, a similar 3-rings based anthraquinone chemotype was already selected with NSC279836, the most active compound of our study. Next, NSC47147 and NSC247562 are pyrrole derivatives composed of 3 successive pyrrole rings and various aliphatic substitutions on one end of the molecule. Looking at the 550+ molecules tested (143 from the oncology set and 420 from the natural products set), we could not find other compound (active or inactive) that could be included in this family of molecules.

Finally, NSC785168, NSC785155, NSC785176 and NSC785165 are aporphine derivatives with various substitutions. NSC785168 represents the simplest molecule with NSC785155 having additional methoxyl groups and a dioxolane ring, while NSC785165 and NSC785176 harbor a dibenzylamine substitution. Moreover, NSC785165 and 227 NSC785176 only differ by their formulation, being mono- or di-hydrochloric acids, respectively. Accordingly, only one of them has been kept for further analysis, NSC785176.

NSC785154, NSC785178, NSC785158, NSC785171 and NSC785160 were inhibitors to a certain level with a residual IN-GCN2 interaction signal of 16.4%, 16.7%, 25%, 25.3% and 29.9%, respectively. NSC785159, NSC785170 and NSC36351 were inactive (within 10% of the DMSO control) while NSC266535 induced a slight stimulation of the interaction (39% increase in signal).

Altogether, four main chemotypes could be selected with tetracyclines (NSC18334), anthracenes (NSC345647, NSC248605), aporphines (NSC785168, NSC785155, NSC785176, NSC785165) and pyrroles (NSC47147, NSC247562). Accordingly, a homology search was performed in the PubChem database (substructure key-based 2D Tanimoto similarity) to look for derivatives of the 8 molecules identified (except for NSC785165) along with NSC279836.

### Example 3: Structure activity relationship studies

The mode of action of the molecules of the present invention on the IN-GCN2 interaction was studied by testing the lead compound in each series - anthracenes, tetracyclines and aporphines, for its activity on an in vitro phosphorylation assay. NSC345647 for anthraquinones; NSC18334 for tetracyclines and NSC785155 for aporphines.

### Example 3.1 Tetracyclines

Starting from NSC18334 as a lead compound, 18 derivatives listed in the following Table 4 have been identified and were available through the DTP.

**Table 4**

| | |
|---|---|
| IA-1a | 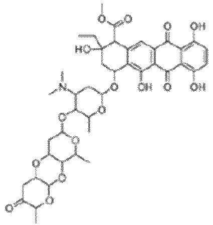 |
| NSC18334 | |
| IA-1b | 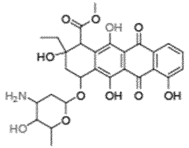 |
| NSC263854 | |
| IA-1c | 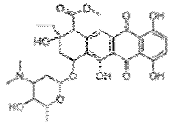 |
| NSC267229 | |
| IA-1d | 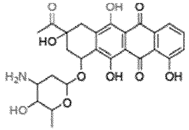 |
| NSC180024 | |
| IA-1e | 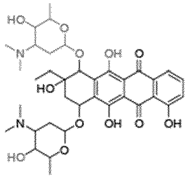 |
| NSC136044 | |
| IA-1f | 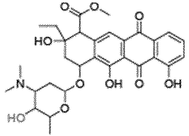 |
| NSC100290 | |
| IA-1g | 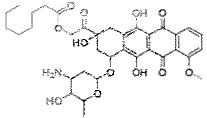 |
| NSC149584 | |
| IA-1h | 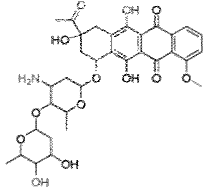 |
| NSC349631 | |
| IA-1i | 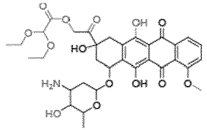 |
| NSC292652 | |
| IA-1j | 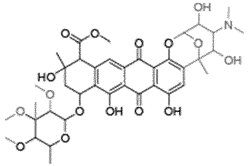 |
| NSC70845 | |
| IA-1k | 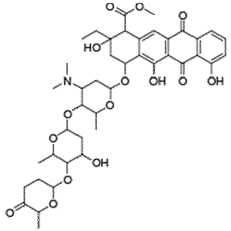 |
| NSC208734 | |
| IA-1l | 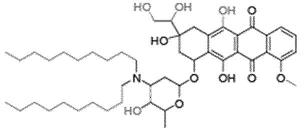 |
| NSC292686 | |
| IA-1m | 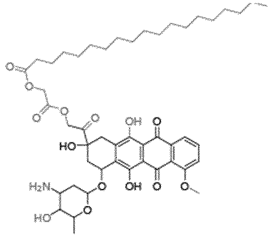 |
| NSC261057 | |
| IA-1n | 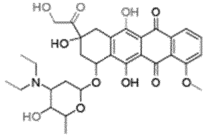 |
| NSC268239 | |
| IA-1o | 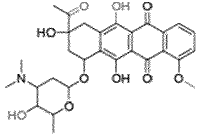 |
| NSC258812 | |
| IA-1p | 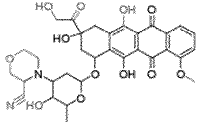 |
| NSC357704 | |
| IA-1q | 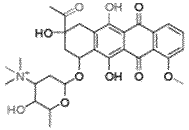 |
| NSC245426 | |
| IA-1r | 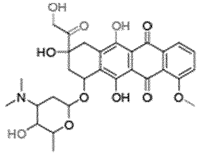 |
| NSC261045 | |

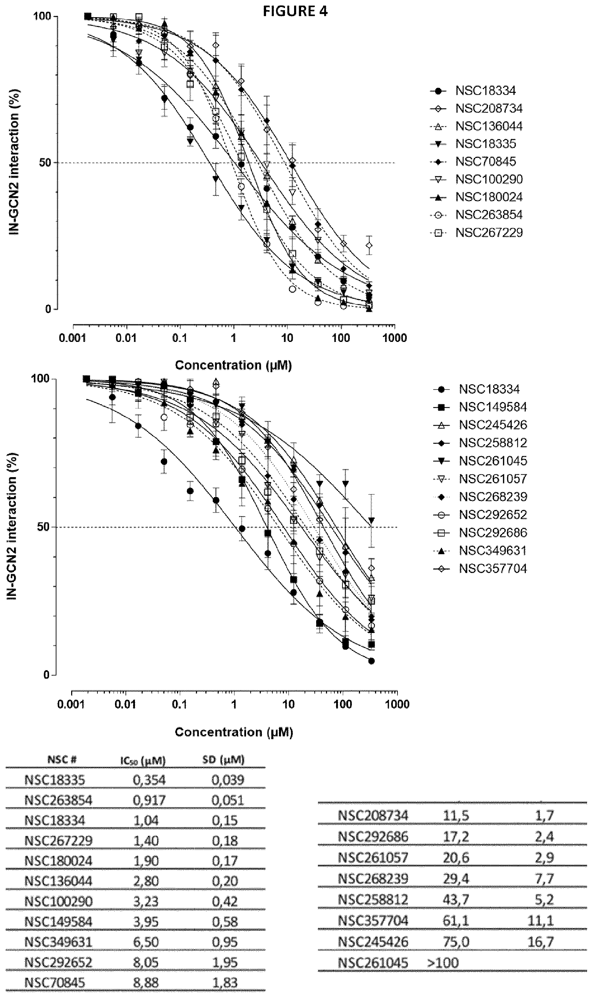

Fresh dilutions of all 19 molecules from powder vials were tested in the IN-GCN2 interaction assay and the corresponding IC50 values and standard deviations were calculated (Figure 4). NSC18334 had a similar activity (1.04 µM +/- 0.15 250 µM) compared to the plated compounds used in the screen (790 nM +/- 45 nM, Figure 1). Interestingly, the derivatives exhibited a wide range of activity, with IC50s from >300 µM to as low as 350 nM.

Compounds IA-1a (NSC18335) and IA-1c (NSC267229) harbor the same tetracycline core as seen in NSC18334. Differences arise from the THP chain, with IA-1a having an extra bond closing a ring in between the THP 2 and 3 while 1c presents a single THP. Compound IA-1a was the most active compound with an IC50 of 354 nM +/- 39 nM, corresponding to a 2.9-fold increase in activity compared to the parental compound NSC18334. IA-1c was slightly less active than NSC18334 with an IC50 of 1.4 µM +/- 0.2 µM. This seems to indicate that only one THP group is sufficient to inhibit the IN-GCN2 interaction but locking the orientation of longer THP chain into a planar conformation could be beneficial. In addition, NSC265211 was related to NSC18334 with the presence of two hydroxyl groups on the third THP of the chain where NSC18334 has a carbonyl group. The limited activity of NSC265211 compared to NSC18334 (about 60% inhibition at 100µM) highlights the important role of this third THP group to engage specific interactions with the target.

Compound IA-1j (NSC70845) harbors an extra bicyclic THP substitution fused on ring D of the tetracyclic moiety. The ethyl group of ring A is replaced by a methyl group. In addition, the single THP substituent on ring A (similarly to IA-c) is substituted with 2 methyl and 3 methoxyl groups. This compound was still active in the IN-GCN2 interaction assay but with an IC50 of 8.9 µM +/- 1.8 µM corresponding to an 8-fold decrease in potency compared to NSC18334 and 6-fold compared to IA-1c. Interestingly, NSC86005 included in the natural products set harbored a similar modification of ring D but lacked any substituent on ring A (THP absent). Even if it was only screened at a single dose, we found it similarly active against the IN-GCN2 interaction compared to IA-1j (around 86% inhibition at 100 µM). This tends to indicate that the presence of a single THP is not required for activity when in the presence of a bicyclical THP modification on ring D.

Removing a hydroxyl group on ring D of NSC18334 leads to compounds IA-1k (NSC208734) exhibiting an IC50 of 11.5 µM +/- 1.7 µM. This corresponded to an 11-fold decrease in activity compared to NSC18334, highlighting the importance of this substitution in the activity. However, a similar variation but in the context of IA-1e led to a more mitigated result. Compound IA-1f (NSC100290) had an IC50 value of 3.2 µM +/-0.4 µM corresponding to only a 2-fold decrease in activity compared to IA-c. Thus, it appeared that this hydroxyl group on ring D played a crucial role in the activity depending on the length of the THP chain.

Compounds IA-1b (NSC263854), IA-1d (NSC180024) and IA-1e (NSC136044) were derivatives also lacking the hydroxyl group on ring D but with an extra hydroxyl group on ring B. All three molecules were active with IC50 values of 0.92 µM +/- 0.05 µM, 1.9 µM +/- 0.17 µM and 2.8 µM +/- 0.2 µM, respectively. Accordingly, IA-1b is slightly more active than 1c and in the range of NSC18334. Because IA-1b lacks the dimethylamine substituent on the THP chain, we could not ascertain the importance of presence of the extra hydroxyl group on ring B. However, compounds IA-1g (NSC149584), IA-1h (NSC349631), IA-1i (NSC292652), IA-1l (NSC292686), IA-1m (NSC261057), IA-1n (NSC268239), IA-1o (NSC258812), IA-1p (NSC357704), IA-1q (NSC245426) and IA-1r (NSC261045) all harbored this hydroxyl arrangement seen in IA-1b/ IA-1d/ IA-1e but having a methoxyl group on ring D. They all exhibited a substantial increase in IC50 with values ranging from 4 µM for IA-1g to >300 µM for IA-1r. NSC82151 and NSC256439 inhibited at similar level the IN-GCN2 interaction when tested at single dose (around 70% inhibition at 100 µM). In fact, these two compounds derive from IA-1d by the presence of a methoxyl group or a hydrogen in place of the hydroxyl on ring D. Thus while the two molecules are less active than IA-1d (~70% inhibition at 4 µM), it seems that the presence of an hydroxyl is important in that position, either to create an H-bond with the target or to increase electron mobility throughout the tetracycline moiety.

### Example 3.2 Anthracenes

In the case of anthracenes, compounds similar to NSC345647 and NSC248605 listed in the following table 5 were tested.

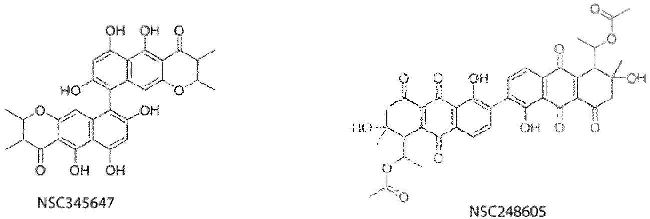

**Table 5**

| | |
|---|---|
| I-2a | 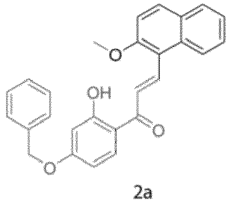 |
| NSC58446 | |
| I-2b | 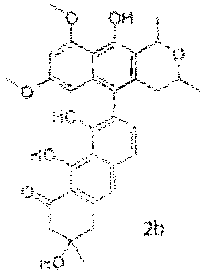 |
| NSC235814 | |
| I-2c | 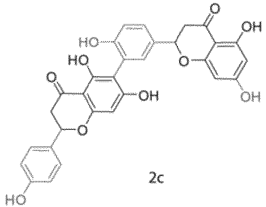 |
| NSC339191 | |

Fresh dilutions of NSC345647 and NSC248605- exhibited better activities than that observed during the initial screen from a plated source.

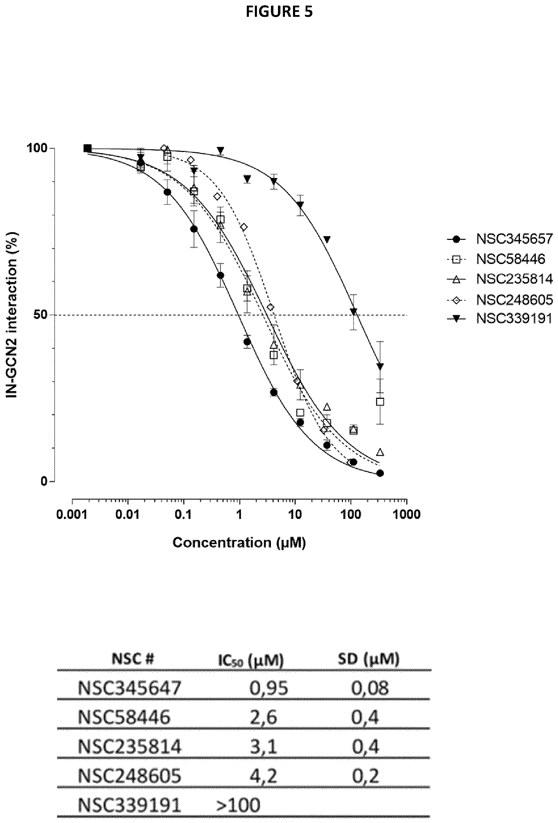

They inhibited the IN-GCN2 interaction with an IC50 value of 0.95 µM +/- 0.08 µM and 4.2 µM +/- 0.2 µM, respectively (Figure 5). This corresponded to a 5- to 6- fold increase in potency. This apparent discrepancy may be due to a problem with the screen plate (prepared directly by the DTP) or a stability issue for the compounds. Still, derivatives I-2a (NSC58446) and I-2b (NSC235814) were found active with IC50 values in the single digit micromolar range (2.6 µM +/- 0.4 µM, 3.1 µM +/- 0.4 µM, respectively) while I-2c (NSC339191) was almost inactive (>100µM). Because of the limited number of molecules, no structure-activity relationship could be determined. Nonetheless, looking only at NSC345647, NSC248605 and I-2b that are the three most alike molecules, it seems that the orientation of the 2 tricycles is not crucial in the overall activity of the molecule. While more in-depth analysis would be required to better understand the mechanism of action, the constraint inherent with this single bond linker might indicate that only one tricyclic
moiety is used to bind its target.

The following derivatives have been identified as listed in the following Table 6.

**Table 6:**

| | |
|---|---|
| I-2a | 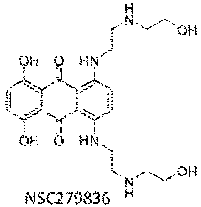 |
| NSC279836 | |
| I-2d | 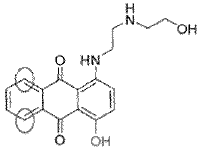 |
| NSC299187 | |
| I-2e | 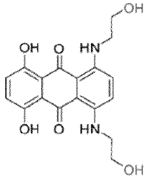 |
| NSC227207 | |
| I-2f | 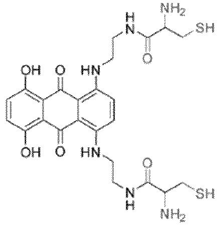 |
| NSC645018 | |
| I-2g | 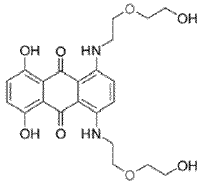 |
| NSC339683 | |
| I-2h | 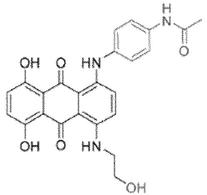 |
| NSC125898 | |
| I-2i | 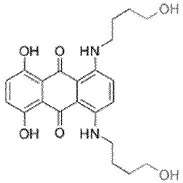 |
| NSC317016 | |
| I-2j | 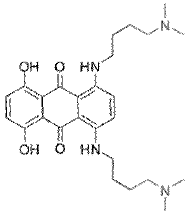 |
| NSC186848 | |
| I-2k | 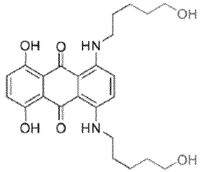 |
| NSC321458 | |
| I-2l | 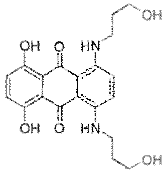 |
| NSC317017 | |
| I-2m | 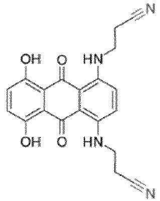 |
| NSC128432 | |
| I-2n | |
| NSC179818 | |

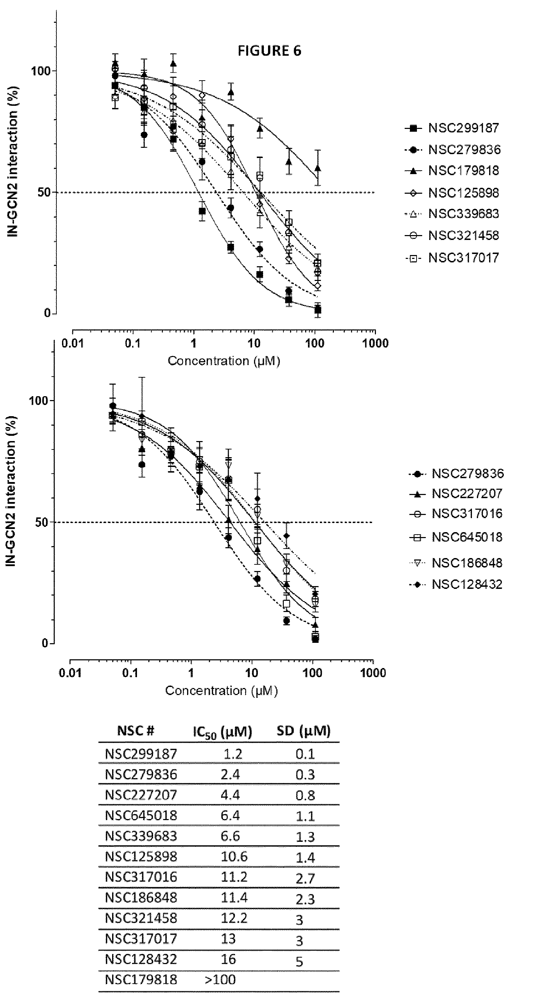

Compound I-2n (NSC179818) is an acridine derivative that represented a simple version of the tricyclic core of NSC279836. The limited activity observed with I-2n (IC50 >100 µM) highlight the fact that the electron density and mobility of the anthracene core might be important along with specific substitution to reach additional contact with the target. Interestingly, all other derivatives differed from NSC279836 only by the length and nature of the two aliphatic arms. Compounds I-2e (NSC227207), I-2f (NSC645018), I-2g (NSC339683), I-2h (NSC125898); I-2i (NSC317016), I-2j (NSC186848), I-2k (NSC321458), I-2l (NSC317017) and I-2m (NSC128432) were active in the low micromolar range with IC50 values ranging from 4.4µM to 16.4 µM 342
(Figure 6). The most striking difference relates to the position of the H-bond donor/acceptor along the aliphatic chain. While they all presented a secondary amine at position 1, it appeared important to have either a secondary amine or alcohol at position 4 and to a lesser extend position 7. This is also the case with compound I-2d exhibiting increased activity compared to NSC279836 with an IC50 value of 1.21 µM +/-0.12µM compared to 2.39 µM +/- 0.35 µM. Even if this 2-fold decrease in IC50 was moderate, it indicated that only one arm is sufficient for the activity of the molecule. As seen with the tetracyclines, the absence of two hydroxyl groups on ring A compared to NSC279836 may also be detrimental to the overall activity of I-2d.

### Example 3.3 Aporphines

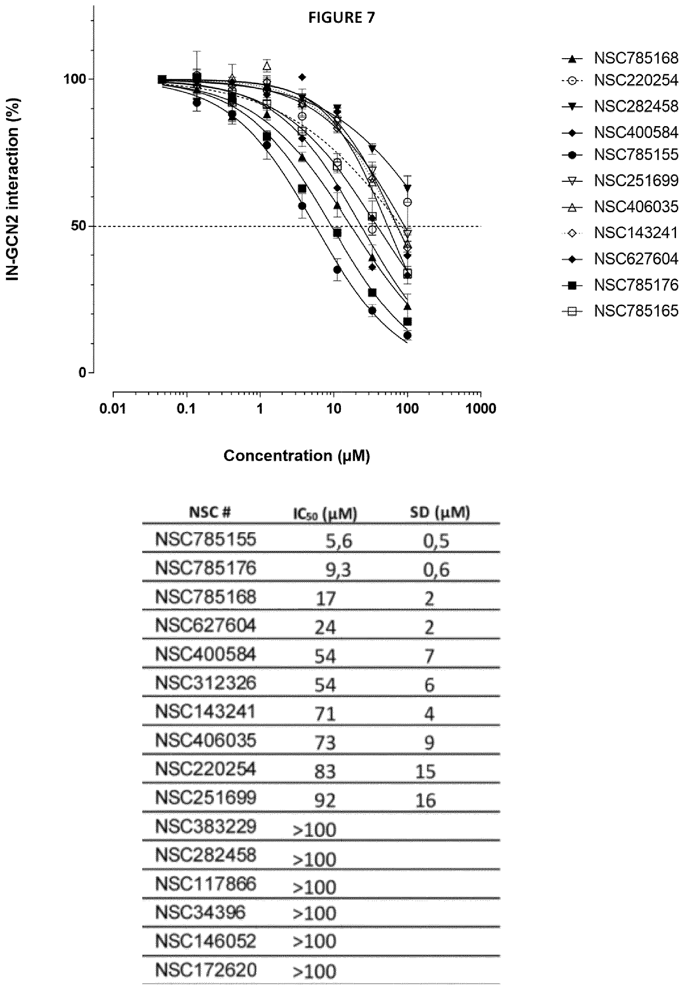

Along the three selected inhibitors, NSC785155, NSC785176 and NSC785168, 13 derivatives listed in the following Table 7 were tested in the IN-GCN2 interaction assay (Figure 7).

**Table 7**

| | |
|---|---|
| IIIa | 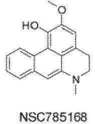 |
| NSC785168 | |
| IIIb | 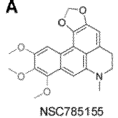 |
| NSC785155 | |
| IIIc | 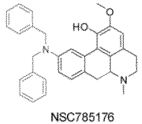 |
| NSC758176 | |
| III-3a | 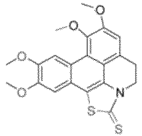 |
| NSC627604 | |
| III-3c | 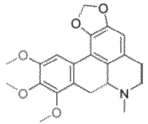 |
| NSC312326 | |
| III-3d | 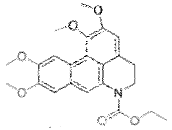 |
| NSC143241 | |
| III-3e | 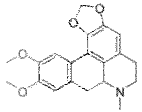 |
| NSC406035 | |
| III-3f | 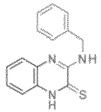 |
| NSC220254 | |
| III-3g | 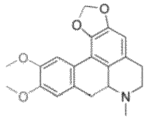 |
| NSC251699 | |
| III-3h | 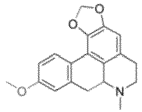 |
| NSC383229 | |
| III-3i | 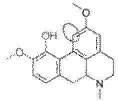 |
| NSC282458 | |
| III-3j | 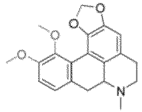 |
| NSC117866 | |
| III-3k | 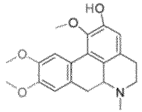 |
| NSC34396 | |
| III-3l | 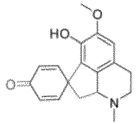 |
| NSC146052 | |
| III-3m | 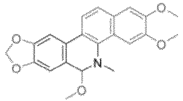 |
| NSC172620 | |

Among those, 6 molecules were either inactive or with undetermined IC50 (>100 µM), namely NSC383229, NSC282458, NSC117866, NSC34396, NSC146052 and NSC172620 (Figure 5B). For the rest of the derivatives, their IC50 values ranged from 24 µM to 92 µM. Accordingly, none were efficient inhibitors of the IN-GCN2 interaction and NSC785155 and NSC785176 were still the most active molecules. The main chemical differences between these two molecules are the closing of a dioxolane ring seen in NSC785155 and the presence of a dibenzylamine substitution on NSC785176. Both probably have a positive impact on the activity of the core structure represented by NSC785168. Indeed, NSC34396 was found inactive against the IN-GCN2 interaction while the dioxolane-containing counterpart 3e exhibited a moderate inhibition (IC50 value of 73 µM +/- 9 µM).

The introduction of a thiazolidine thiocetone on the bottom of the molecule (III-3a, NSC627604) did not seem to improve the activity of the molecule compared to the previously identified NSC785154 (~80% and 93.6% inhibition at 100 µM, respectively). Even, an ethyl carbamate substitution as seen in III-3d (NSC143241) appeared to be detrimental with only ~55% inhibition at 100µM.

When comparing these structures, these molecules appeared different in the planarity. Globally, active compounds are predicted to be planar or only slightly out of plan (due to the piperidine ring) while molecules that are not planar (with distortion induced by the central ring) were less active to inactive. A first example of this possible dependency on global conformation is the better activity observed with compounds
III-3e (NSC406035, S isomer) compared to III-3g (NSC251699, R isomer) that represent stereoisomers. Similarly, the introduction of a single hydrogen atom in the central ring of the active compound NSC785168 is sufficient to abolish the activity of the corresponding derivative (NSC785170). Finally, III-3c (NSC312326) harbors additional hydrogen atoms on the aporphine core compared to NSC785155. This non-planar conformation seemed to be highly detrimental for the activity with a 10-fold decrease in activity.

### Example 4: Efficacy studies as inhibitors of GCN2

The efficacy of the compounds according to the present invention on the IN-GCN2 interaction was evidenced using in vitro phosphorylation assay.

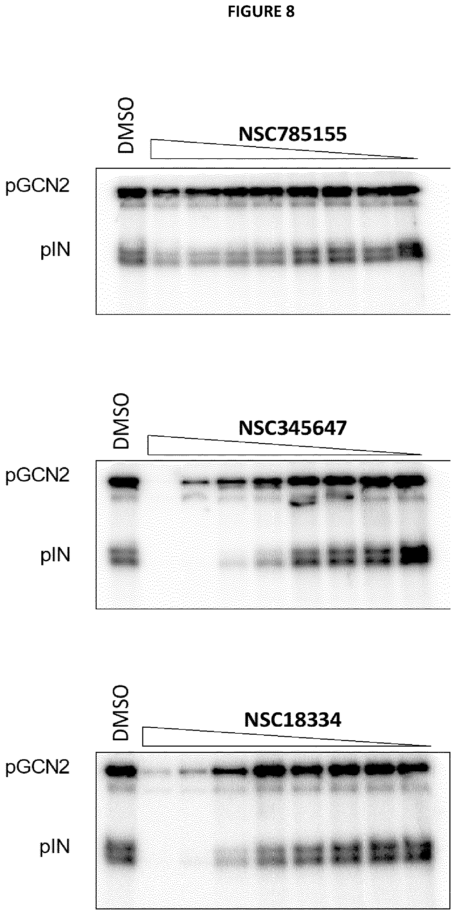

In this assay, GCN2 (25nM) was incubated with IN (700nM) led to the auto phosphorylation of GCN2 (Figure 8, top band) and the phosphorylation of the IN (bottom band). NSC785155 (aporphine) inhibited the phosphorylation of IN and to a lesser extend the autophosphorylation of GCN2 at concentrations similar to that necessary for the inhibition of the IN-GCN2 interaction. Likewise, the inhibition of IN phosphorylation by NSC345647 was also observed at concentrations where the IN-GCN2 interaction was inhibited (submicromolar concentrations). However, a selectivity could be observed with the inhibition of GCN2 auto phosphorylation requiring about 6-times higher concentrations of the anthraquinone. Of note, a total inhibition of the phosphorylation of both IN and GCN2 was observed at the highest concentrations (>1 µM). NSC18334 (tetracycline) exhibited a similar profile than that of NSC345647 with a full inhibition of the phosphorylation of IN and GCN2 at 10 µM (97% and 89% inhibition, respectively, Figure 9). Again, a selectivity for the inhibition of the phosphorylation of IN over GCN2 was observed, requiring about 10-times lower concentration (Figure 9).

## Claims

1. A compound for use as anti-retroviral therapy chosen among a compound having the following general formula (I) or (IA): 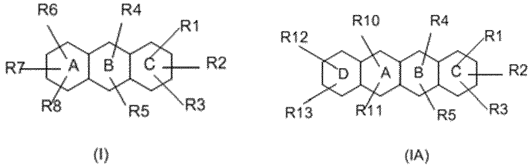
wherein rings A, B, C and D are selected from a six-membered saturated or unsaturated optionally substituted carbocylic ring, optionally containing one to two heteroatoms, selected from O, N or S; wherein R₁, R₂ and R₃ are each independently selected from hydrogen, hydroxy, carbonyl, optionally substituted C₁-C₆ alkyl, a polycyclic saturated or unsaturated ring containing one to two heteroatoms, selected from O, N or S, substituted with one or more substituents independently selected from H, OH, carbonyl, optionally substituted C₁-C₆ alkyl; wherein the alkyl may be substituted with halogen, alkoxy, carbonyl, cyano, NO₂, C(O)OR, or -OC(O)R, wherein R is any group selected from H or C₁-C₆ alkyl; wherein R₄ and R₅ are each independently selected from H, carbonyl or OH;
wherein each of the groups R₆, R₇ and R₈ are independently selected from H, OH, carbonyl, optionally substituted C₁-C₆ alkyl, wherein the alkyl may be substituted with halogen, alkoxy, carbonyl, cyano, NO₂, C(O)OR or -OC(O)R, and R is as defined above; or a polycyclic saturated or unsaturated ring containing C₁₀-C₁₈ ring atoms optionally containing one to two heteroatoms, selected from O, N or S; substituted with one or more substitutions independently selected from H, OH, carbonyl, optionally substituted C₁-C₆ alkyl; wherein each of the groups R₁₀ and R₁₁ are independently selected from H, OH, carbonyl, optionally substituted with C₁-C₆ alkyl, wherein the alkyl may be substituted with halogen, alkoxy, carbonyl, cyano, NO2, C(O)OR or -OC(O)R, and R is as defined above;
wherein each of the groups R₁₂ and R₁₃ are independently selected from H, OH, carbonyl, C₁-C₆ alkoxy, C(O)OR or -OC(O)R, R is as defined above, optionally substituted C₁-C₆ alkyl, wherein the alkyl may be substituted with halogen, alkoxy, carbonyl, cyano, NO₂; or a group of formula (IV): 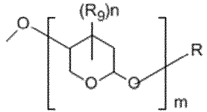
R₉ being selected from H, CO, C₁-C₆ alkyl, N(R)₂, wherein R is as defined above; and n is any integer chosen between 1-4; and m is any integer chosen between 1-4;
or a compound having the following general formula (II): 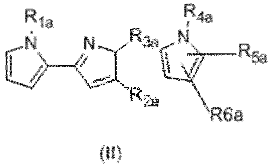
wherein R₁ₐ and R₄ₐ is hydrogen, phenyl, C₁-C₂₀ alkyl or C₂-C₂₀ alkenyl, wherein the alkyl and alkenyl groups are unsubstituted or substituted by 1 to 3 substituents chosen independently from halogen, alkoxy, hydroxy, aryl and aryloxy;
wherein R₂ₐ is hydrogen, C₁-C₆ alkyl, cyano, carboxy or (C₁-C₆ alkoxy) carbonyl;
wherein R₃ₐ is C₂-C₂₀ alkenyl;
wherein each of R₅ₐ and R₆ₐ, may be the same or different, independently represents hydrogen, C₁-C₂₀ alkyl, C₂-C₂₀ alkenyl, C₂-C₂₀ alkanoyl, C₃-C₂₀ alkenoyl, phenyl, wherein the alkanoyl, alkenoyl, alkyl and alkenyl groups are unsubstituted or substituted by 1 to 3 substituents chosen independently from halogen, C₁-C₆ alkoxy, hydroxy, aryl, aryloxy, cyano, carboxy, (C₁-C₆ alkoxy) carbonyl , aryloxy or aryl; or
wherein R₅ₐ and R₆ₐ, taken together with adjacent carbon ring atoms to which they are linked, form a C₄ - C₁₂ completely or partially saturated or unsaturated cyclic ring optionally contains one or more heteroatoms selected from O, S or N which is unsubstituted or substituted by C₁-C₁₂ alkyl, C₂-C₁₂ alkenyl, wherein the alkyl, and alkenyl groups are in turn unsubstituted or substituted by halogen, C₁-C₆ alkoxy, hydroxy, cyano, carboxy, (C₁-C₆ alkoxy) carbonyl, aryloxy or aryl; or a pharmaceutically acceptable salt thereof;
or a compound having the following general formula (III): 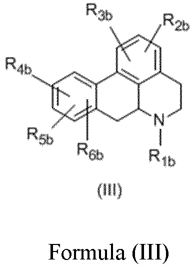
wherein R₁₃ is selected from H, C₁-C₂₀ alkyl, C₂-C₂₀ alkenyl, C₂-C₂₀ alkanoyl, C₃-C₂₀ alkenoyl, phenyl, wherein the alkanoyl, alkenoyl, alkyl and alkenyl groups are unsubstituted or substituted by 1 to 3 substituents chosen independently from halogen, C₁-C₆ alkoxy, hydroxy, aryl, aryloxy, cyano, carboxy, (C₁-C₆ alkoxy) carbonyl, aryloxy or aryl;
wherein each of R_{2b} and R_{3b}, which are the same or different, independently represents H, OH, carbonyl, optionally substituted C₁-C₆ alkyl; wherein the alkyl may be substituted with halogen, alkoxy, carbonyl, cyano, NO2, C(O)OR or -OC(O)R, wherein R is any group selected from H or C₁-C₆ alkyl;
or wherein R_{2b} and R_{3b}, taken together with adjacent carbon ring atoms which they are linked, form a C₄ - C₁₂ completely or partially saturated or unsaturated cyclic ring optionally contains one or more heteroatoms selected from O, S or N which is unsubstituted or substituted by C₁-C₁₂ alkyl, C₂-C₁₂ alkenyl, wherein the alkyl, and alkenyl groups are in turn unsubstituted or substituted by halogen, C₁-C₆ alkoxy, hydroxy, cyano, carboxy, (C₁-C₆ alkoxy) carbonyl, aryloxy or aryl;
wherein each of the groups R₄₆, R_{5b} and R_{6b} are independently selected from H, OH, halogen, C₁-C₆ alkoxy, cyano, carboxy, (C₁-C₆ alkoxy) carbonyl, aryloxy, aryl or NR'R"; R' and R‴ being the same or distinct, independently represents H, OH, carbonyl, optionally substituted C₁-C₆ alkyl, cyano, carboxy, (C₁-C₆ alkoxy) carbonyl, aryloxy or aryl;
or a pharmaceutically acceptable salt thereof.

2. The compound for use according to claim 1, wherein said compound has the formula (I) and wherein the rings A, B, and C in compound of formula (I) correspond to an anthracene ring of formula:

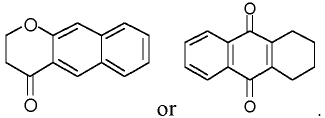

3. The compound for use according to claim 2, wherein said compounds have the following formula: 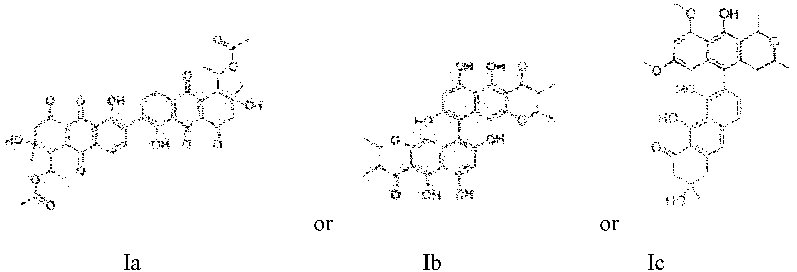 or a pharmaceutically acceptable salt, enantiomer, stereoisomer, or tautomer thereof.

4. The compound for use according to claim 2, wherein said compounds are chosen among:
1,4-dihydroxy-5,8-bis[[2-[(2-hydroxyethyl)amino]ethyl]amino]-9,10-anthracenedione;
1-hydroxy-4-[[2-[(2-hydroxyethyl)amino]ethyl]amino]- 9,10-anthracenedione;
1,8-bis[(2-hydroxyethyl)amino]anthraquinone;
1,4-bis(N-Cysteinyldiethylamino)-5,8-dihydroxy anthraquinone;
1,4-dihydroxy-5,8-bis[[2-(2-hydroxyethoxy)ethyl]amino]- 9,10-Anthracenedione;
N-[4-[[5,8-dihydroxy-4-(2-hydroxyethylamino)-9,10-dioxoanthracen-1-yl]amino]phenyl] acetamide;
1,4-dihydroxy-5,8-bis[(4- hydroxybutyl)amino]-9,10-Anthracenedione;
1,4-bis[[4-(dimethylamino)butyl]amino]-5,8-dihydroxy-9,10-Anthracenedione;
1,4-dihydroxy-5,8-bis(5-hydroxypentylamino)anthracene-9,10-dione;
1,4-dihydroxy-5,8-bis[(3-hydroxypropyl)amino]-9,10-Anthracenedione;
3-[[4-(2-cyanoethylamino)-5,8-dihydroxy-9,10-dioxoanthracen-1-yl]amino]propanenitrile; or
a pharmaceutically acceptable salt, enantiomer, stereoisomer, or tautomer thereof.

5. The compound for use according to claim 1, wherein rings A, B, C, and D in compound of formula (IA) correspond to an anthracycline ring of formula:

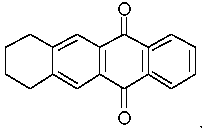

6. The compound for use according to claim 5, wherein said compound is chosen among:
2,3,4,6,11-hexahydro-2,5,7,10-tetrahydroxy-6,11-dioxo-4-[2,3,6-trideoxy-4-O-[2,6-dideoxy-4-O-((2R-trans)-tetrahydro-6-methyl-5-oxo-2H-pyran-2-yl)-.alpha.-L-lyxo-hexopyranosyl]-3-(dimethylamino)-.alpha.-L-lyxo-hexopyranosy-1-Naphthacenecarboxylic acid, or cinerubine A;
4-[(3-amino-2,3,6-trideoxy-α-L-lyxo-hexopyranosyl)oxy]-2-ethyl-1,2,3,4,6,11-hexahydro-2,5,7,12-tetrahydroxy-6,11-dioxo-, methyl ester, hydrochloride, [1R-(1α,2β,4β]-1-naphthacenecarboxylic acid;
2-ethyl-1,2,3,4,6,11-hexahydro-2,5,7,10-tetrahydroxy-6,11-dioxo-4-[[2,3,6-trideoxy-3-(dimethylamino)-α-L-lyxo-hexopyranosyl]oxy]-, methyl ester, [1R-(1α,2β,4β)]-1-naphthacenecarboxylic acid;
8-acetyl-10-[[3-amino-2,3,6-trideoxy-α-L-lyxo-hexopyranosyl]oxy]-7,8,9,10-tetrahydro-1,6,8,11-tetrahydroxy-, (8S-cis)-1-naphthacenecarboxylic acid;
Rhodomycin A;
Aklavin;
2-[4-[(3-amino-2,3,6-trideoxy-α-L-lyxo-hexopyranosyl)oxy]-1,2,3,4,6,11-hexahydro-2,5,12-trihydroxy-7-methoxy-6,11-dioxo-2-naphthacenyl]-2-oxoethyl ester, hydrochloride, (2S-cis)- octanoic acid;
8-acetyl-10-[[3-amino-2,3,6-trideoxy-4-O-(2,6-dideoxy-.alpha.-L-lyxo-hexopyranosyl)-α-L-lyxo-hexopyranosyl]oxy]-7,8,9,10-tetrahydro-6,8,11-trihydroxy-1-methoxy-, (8S-cis)- 5,12-naphthacenedione;
Diethoxy-,2-[4-[(3-amino-2,3,6-trideoxy-.α.-L-lyxo-hexopyranosyl) oxy]-1,2,3,4,6,11-hexahydro-2,5,12-trihydroxy-7-methoxy-6,11-dioxo-2-naphthacenyl]-2-oxoethyl ester, hydrochloride, (2S-cis)- acetic acid; Nogalamycin;
Aclacinomycin A;
7-[4-(didecylamino)-5-hydroxy-6-methyloxan-2-yl]oxy-9-(1,2-dihydroxyethyl)-6,9,11-trihydroxy-4-methoxy-8,10-dihydro-7H-tetracene-5,12-dione;hydrochloride;
2-[2-[4-[(3-amino-2,3,6-trideoxy-α-L-lyxo-hexopyranosyl)oxy]-1,2,3,4,6,11-hexahydro-2,5,12-trihydroxy-7-methoxy-6,11-dioxo-2-naphthacenyl]-2-oxoethoxy]-2-oxoethyl ester, hydrochloride, (2S-cis)- octadecanoic acid;
7,8,9,10-tetrahydro-6,8,11-trihydroxy-8-(hydroxyacetyl)-1-methoxy-10-[[2,3,6-trideoxy-3-(diethyl amino)-α-L-lyxo-hexopyranosyl]oxy]-, hydrochloride, (8S-cis)- 5,12-naphthacenedione;
10-[[3-(3-cyano-4-morpholinyl)-2,3,6-trideoxy-α-L-lyxo-hexopyranosyl]oxy]-7,8,9,10-tetrahydro-6,8,11-trihydroxy-8-(hydroxyacetyl)-1-methoxy-, (8S-cis)- 5,12-naphthacenedione;
3-Acetyl-3,5,12-trihydroxy-10-methoxy-6,11-dioxo-1,2,3,4,6,11-hexahydrotetracen-1-yl-2,3,6-trideoxy-3-(trimethylazaniumyl)hexopyranoside chloride;
7,8,9,10-tetrahydro-6,8,11-trihydroxy-8-(hydroxyacetyl)-1-methoxy-10-[[2,3,6-trideoxy-3-(dimethyl lamino)-α-L-lyxo-hexopyranosyl] oxy]-, hydrochloride, (8S-cis)- 5,12-naphthacenedione; or
a pharmaceutically acceptable salt, enantiomer, stereoisomer, or tautomer thereof.

7. The compound for use according to claim 1, wherein said compound has the formula (II) is chosen among : 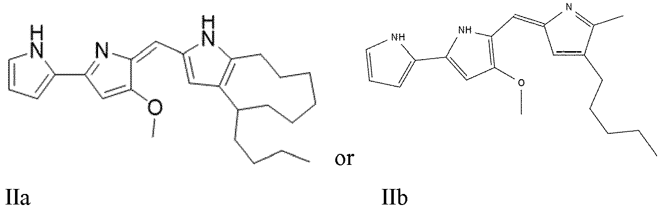 or pharmaceutically acceptable salt, enantiomer, stereoisomer, or tautomer thereof.

8. The compound for use according to claim 1, wherein said compound has the general formula (III) and is chosen among the following compounds:
hydroxy-2-methoxydehydroaporphine hydrobromide;
dehydroocopodine;
tranexamic acid;
7,8,10,11-Tetramethoxy-4,5-dihydrodibenzo[de,g] [1,3]thiazolo[5,4,3-ij]quinoline-2-thione;
Ocopodine (+);
Dibenzo[de,g]quinoline-6-carboxylic acid, 4,5-dihydro- 1,2,9,10-tetramethoxy-, ethyl ester;
Eximine;
2-benzylamino-3-mercaptoquinoxaline;
Dicentrine (-);
5H-Benzo[g]-1,3-benzodioxolo[6,5,4-de]quinoline, 6,7,7a,8-tetrahydro-10-methoxy-7-methyl;
2,10-dimethoxy-6-methyl-5,6,6a,7-tetrahydro-4H-dibenzo[de,g] quinolin-11-ol;
6,7,7a,8-tetrahydro-11,12-dimethoxy-7-methyl-, (S)-bulbocapnine, O-methyl- 5H-benzo[g]-1,3-benzodioxolo[6,5,4-de]quinoline;
5,6,6a,7-tetrahydro-1,2,9,10-tetramethoxy-6-methyl-,(S)-6aα-aporphine, 1,2,9,10-tetramethoxy- 4H-dibenzo[de,g]quinoline;
2',3',8',8'a-tetrahydro-6'-hydroxy-5'-methoxy-1'-methyl-spiro[2,5-cyclohexadiene-1,7'(1'H)-cyclopent[ij]isoquinolin]-4-one;
5,6-dihydro-2,3,6-trimethoxy-5-methyl-benzo[c][1,3]dioxolo[4,5-j] phenanthridine; or
or an acceptable salt, enantiomer, stereoisomer, or tautomer thereof.

9. A pharmaceutical or veterinary composition comprising a compound according to any one of claims 1 to 8, or an acceptable salt thereof, and a pharmaceutically or veterinary acceptable carrier or excipient.

10. The pharmaceutical or veterinary composition according to claim 9, wherein the carrier is selected from saline, Cremophor EL, propylene glycol, polyethylene glycol and alcohol.

11. The pharmaceutical or veterinary composition according to claim 9 or 10, wherein the composition is present in a form suitable for oral, aerosol, parenteral, subcutaneous, intravenous, intraarterial, intramuscular, intraperitoneal, rectal, and/or vaginal administration.

12. The compound or composition according to any one of the preceding claims for use in a method of treating and/or preventing retroviral infection in a mammal in need thereof, comprising administering to said mammal a therapeutically effective amount of said compound or composition to said mammal.

13. The compound or composition for use in a method of claim 12, wherein said retroviral infection is caused by a retrovirus chosen among HIV-1/2, human T-cell lymphotropic virus (HTLV), feline leukemia virus, feline immunodeficiency virus, coronaviruses such as SARS-CoV-1/2, MERS-CoV or novel emerging coronaviruses, flaviviruses such as Hepatitis C virus, Zika, Dengue 1-4, Usutu, West Nile, Baiyangdian virus (BYDV) or other vector-borne orthoflaviviruses.

14. The compound or composition for use in a method of claim 12 or 13, further comprising use of one or more anti-viral agent.

15. The compound or composition for use in a method of claim 14, wherein the anti-viral agent is chosen among saquinavir, indinavir, ritonavir, nelfinavir, atazanavir, darunavir, fosamprenavir, tipranavir and amprenavir; nucleoside reverse transcriptase inhibitors including but not limited to, zidovudine, didanosine, stavudine, lamivudine, zalcitabine, emtricitabine, tenofovir disoproxil fumarate, AVX754 and abacavir; non-nucleoside reverse transcriptase inhibitors including, but not limited to, nevaripine, delavirdine, calanolide A, TMC125 and efavirenz; combination drugs including, but not limited to, efavirenz/emtricitabine/tenofovir disoproxil fumarate, lamivudine/zidovudine, abacavir/lamivudine, abacavir/lamivudine/zidovudine, emtricitabine/tenofovir disoproxil fumarate, sulfamethoxazole/trimethoprim, and lopinavir/ritonavir; entry and fusion inhibitors, including, but not limited to, enfuvirtide, AMD070, BMS-488043, fozivudine tidoxil, GSK-873,140, PRO 140, PRO 542, Peptide T, SCH-D, TNX-355, and UK-427,857; treatments for opportunistic infections and other conditions associated with AIDS and HIV including, but not limited to, acyclovir, adefovir dipivoxil, aldesleukin, amphotericin b, azithromycin, calcium hydroxylapatite, clarithromycin, doxorubicin, dronabinol, entecavir, epoetin alfa, etoposide, fluconazole, ganciclovir, immunoglobulins, interferon alfa-2, ionomycine, isoniazid, itraconazole, megestrol, paclitaxel, peginterferon alfa-2, pentamidine, poly- 1 -lactic acid, ribavirin, rifabutin, rifampin, somatropin, testosterone, trimetrexate, and valganciclovir; integrase inhibitors including, but not limited to, GS 9137, MK-0518; microbicides, including, but not limited to, BMS-378806, C31G, carbopol 974P, carrageenan, cellulose sulfate, cyanovirin-N, dextran sulfate, hydroxyethyl cellulose, PRO 2000, SPL7013, tenofovir, and UC-781, and IL- 2.
